# EUROPEAN PATENT APPLICATION

(11) **EP 4 321 170 A1**
(43) Date of publication of application: **14.02.2024**
(21) Application number: 22785037.7
(22) Date of filing: 11.04.2022
(51) Int. Cl.: A61K 38/26, A61K 47/68, A61P 13/12, C07K 14/605

(54) **PHARMACEUTICAL COMPOSITION FOR PREVENTING OR TREATING CHRONIC KIDNEY DISEASE CONTAINING GLUCAGON DERIVATIVE**

(30) Priority: 09.04.2021 KR 20210046648
(71) Applicant: Hanmi Pharm. Co., Ltd., Hwaseong-si, Gyeonggi-do 18536 (KR)
(72) Inventor: LEE, Seon Myeong, Hwaseong-si, Gyeonggi-do 18469 (KR); LEE, Jong Suk, Hwaseong-si, Gyeonggi-do 18469 (KR); KIM, Jung Kuk, Hwaseong-si, Gyeonggi-do 18469 (KR); PARK, Eun Jin, Hwaseong-si, Gyeonggi-do 18469 (KR)
(74) Representative: Mewburn Ellis LLP
(86) International application number: PCT/KR2022/005188
(87) International publication number: WO 2022/216129

(57) **Abstract**

Provided are a glucagon derivative and use thereof.

## Description

### [Technical Field]

The present disclosure relates to a composition including a glucagon derivative, and use thereof.

### [Background Art]

Glucagon is produced and released from the pancreas when blood glucose levels drop due to various causes such as medications, diseases, hormone or enzyme deficiencies, *etc.* It is known that the released glucagon acts on the liver to break down glycogen and to induce the release of glucose, eventually increasing blood glucose levels to a normal level. It is also known that glucagon inhibits hepatic lipid synthesis and also promotes fatty acid combustion to lower the blood lipid levels. In addition, glucagon is known to act directly or indirectly on white fat to induce fat burning and fat browning, resulting in effective weight loss (Nat Rev Endocrinol. 11, 329-38 (2015); Physiol Rev. 97, 721-66 (2017)). This glucagon acts by acting on glucagon receptors.

Recently, as the need has emerged for glucagon derivatives with improved physical properties while acting on glucagon receptors in order to increase efficacy or to improve side effects, the present inventors have developed glucagon derivatives and conjugates thereof acting on glucagon receptors (WO 2018/004283).

The kidneys are located below the diaphragm and behind the stomach, and are organs that remove waste from the body and regulate the amounts of water and salts, electrolytes, and acid-base balance. One of diseases caused by kidney damage or dysfunction is chronic renal disease.

Chronic renal disease, also called chronic kidney disease, is a disease in which proteins are continuously excreted in the urine (proteinuria) or kidney function is deteriorated. In particular, it is known that diabetic or hypertensive patients are at high risk of developing chronic renal disease. In patients with chronic renal disease, waste accumulates in the body due to impaired kidney function, resulting in high blood pressure, anemia, weakened bones, nerve damage, *etc.,* and an increased risk of cardiovascular disease.

Such chronic renal disease is a progressive disease and is difficult to recognize in the early stages, and therefore, early diagnosis through examination is important. When the treatment time is delayed, it progresses to chronic renal failure, requiring dialysis or a kidney transplant.

Treatment of chronic renal disease consists of reducing risk factors and maintaining or restoring kidney function. In particular, hypertension is closely related to chronic renal disease, as it is known that in 60% of patients, chronic renal disease is accompanied by high blood pressure. Therefore, studies are being conducted on treatment of chronic renal disease using antihypertensive drugs, and treatment methods of using angiotensin converting enzyme inhibitors, angiotensin receptor blockers, *etc.* are being studied. However, upon administration of known antihypertensive drugs, side effects such as abnormal renal function, hyperkalemia, and ischemic renal injury have also been reported.

Accordingly, efforts have been made to develop new therapeutic agents and therapies for chronic renal disease by lowering blood pressure and restoring renal function.

### [Disclosure]

### [Technical Problem]

It is necessary that new therapeutic agents and therapies be developed for chronic renal disease by lowering blood pressure and restoring renal function.

### [Technical Solution]

An object of the present disclosure is to provide a pharmaceutical composition for preventing or treating chronic renal disease (chronic kidney disease), the pharmaceutical composition including a glucagon derivative or a conjugate thereof.

Another object of the present disclosure is to provide a method of preventing or treating chronic renal disease, the method including the step of administering the glucagon derivative or conjugate thereof; or the composition including the same.

Still another object of the present disclosure is to provide use of the glucagon derivative or conjugate thereof, or the composition including the same in the prevention or treatment of chronic renal disease.

Still another object of the present disclosure is to provide use of the glucagon derivative or conjugate thereof, or the composition including the same in the preparation of a medicament for use in the prevention or treatment of chronic renal disease.

Still another object of the present disclosure is to provide a pharmaceutical composition for preventing or treating chronic renal disease (chronic kidney disease), the pharmaceutical composition including the glucagon derivative or conjugate thereof; and a GLP-1 receptor agonist or conjugate thereof.

Still another object of the present disclosure is to provide therapeutic use of combination of the glucagon derivative or conjugate thereof; and the GLP-1 receptor agonist or conjugate thereof.

### [Advantageous Effects]

Since a glucagon derivative of the present disclosure exhibits effects of lowering the blood pressure and restoring the renal function, it may be used as a therapeutic agent for patients with chronic renal disease, and additional effects may be expected by using the glucagon derivative in combination with a GLP-1 receptor agonist.

### [Description of Drawings]

FIG. 1 shows the effect of reducing urinary albumin excretion by a long-acting conjugate of a glucagon derivative of SEQ ID NO: 37 of the present disclosure in spontaneously hypertensive rat (SHR) models;
FIG. 2 shows the effect of lowering the systolic (left) and diastolic (right) blood pressures by the long-acting conjugate of the glucagon derivative of SEQ ID NO: 37 of the present disclosure in spontaneously hypertensive rat (SHR) models; and
FIG. 3 shows changes in urine albumin-to-creatinine ratio by combined administration of the long-acting conjugate of the glucagon derivative of SEQ ID NO: 37 of the present disclosure and a long-acting conjugate of CA exendin-4 in spontaneously hypertensive rat (SHR) models.

### [Best Mode]

An aspect of the present disclosure provides a composition including a glucagon derivative or a conjugate thereof. The conjugate refers to a substance in which a peptide having glucagon derivative activity is covalently linked to an immunoglobulin Fc region via a linker.

The composition according to one specific embodiment is a pharmaceutical composition for preventing or treating chronic renal disease (chronic kidney disease), the pharmaceutical composition including a glucagon derivative, or a conjugate thereof.

The composition according to another specific embodiment is characterized in that the glucagon derivative is a peptide including an amino acid sequence of the following General Formula 1: 
X1-X2-QGTF-X7-SD-X10-S-X12-X13-X14-X15-X16-X17-X18-X19-
X20-X21-F-X23-X24-W-L-X27-X28-X29-X30 (General Formula 1, SEQ ID
NO: 46)
 X1-X2-QGTF-X7-SD-X10-S-X12-X13-X14-X15-X16-X17-X18-X19-X20-X21-F-X23-X24-W-L-X27-X28-X29-X30 (General Formula 1, SEQ ID NO: 46)
wherein X1 is tyrosine (Y);
X2 is *alpha*-methyl-glutamic acid (α-methyl-glutamic acid), aminoisobutyric acid (Aib), D-alanine, glycine (G), N-methylglycine (Sar), serine (S), or D-serine;
X7 is threonine (T), valine (V), or cysteine (C);
X10 is tyrosine (Y) or cysteine (C);
X12 is lysine (K) or cysteine (C);
X13 is tyrosine (Y) or cysteine (C);
X14 is leucine (L) or cysteine (C);
X15 is aspartic acid (D), glutamic acid (E), or cysteine (C);
X16 is glutamic acid (E), aspartic acid (D), serine (S), *alpha*-methyl-glutamic acid, or cysteine (C), or is absent;
X17 is aspartic acid (D), glutamine (Q), glutamic acid (E), lysine (K), arginine (R), serine (S), cysteine (C), or valine (V), or is absent;
X18 is alanine (A), aspartic acid (D), glutamic acid (E), arginine (R), valine (V), or cysteine (C), or is absent;
X19 is alanine (A), arginine (R), serine (S), valine (V), or cysteine (C), or is absent;
X20 is lysine (K), histidine (H), glutamine (Q), aspartic acid (D), arginine (R), alpha-methyl-glutamic acid, or cysteine (C), or is absent;
X21 is aspartic acid (D), glutamic acid (E), leucine (L), valine (V), or cysteine (C), or is absent;
X23 is isoleucine (I), valine (V), or arginine (R), or is absent;
X24 is valine (V), arginine (R), alanine (A), cysteine (C), glutamic acid (E), lysine (K), glutamine (Q), *alpha*-methyl-glutamic acid, or leucine (L), or is absent;
X27 is isoleucine (I), valine (V), alanine (A), lysine (K), methionine (M), glutamine (Q), or arginine (R), or is absent;
X28 is glutamine (Q), lysine (K), asparagine (N), or arginine (R), or is absent;
X29 is threonine (T); and
X30 is cysteine (C), or is absent
(with the proviso that when the amino acid sequence of General Formula 1 is identical to SEQ ID NO: 1 or SEQ ID NO: 12, it is excluded).

The composition according to another specific embodiment is characterized in that the glucagon derivative includes any one amino acid sequence selected from the group consisting of SEQ ID NOS: 2 to 11, and 13 to 45.

In the composition according to any one of the previous specific embodiments, the peptide is in the form of a long-acting conjugate, wherein the long-acting conjugate is represented by Formula 1 below:

[Formula 1] X-L-F

wherein X represents a peptide including the amino acid sequence of General Formula 1;
L represents a linker containing ethylene glycol repeating units;
F represents an immunoglobulin Fc region; and
- represents covalent linkages between X and L and between L and F, respectively.

In the composition according to any one of the previous specific embodiments, in General Formula 1,
X2 is aminoisobutyric acid (Aib);
X7 is threonine (T), valine (V), or cysteine (C);
X10 is tyrosine (Y);
X12 is lysine (K);
X13 is tyrosine (Y);
X14 is leucine (L) or cysteine (C);
X15 is aspartic acid (D);
X16 is glutamic acid (E) or serine (S);
X17 is lysine (K), arginine (R), or cysteine (C);
X18 is arginine (R);
X19 is alanine (A) or cysteine (C);
X20 is glutamine (Q) or lysine (K);
X21 is aspartic acid (D) or glutamic acid (E);
X23 is valine (V);
X24 is glutamine (Q);
X27 is methionine (M);
X28 is asparagine (N);
X29 is threonine (T); and
X30 is cysteine (C) or is absent.

The composition according to any one of the previous specific embodiments is characterized in that the peptide includes an amino acid sequence selected from the group consisting of SEQ ID NOS: 7 to 11, and 13 to 25, 27, 29, 31, 33, 35 to 45.

The composition according to any one of the previous specific embodiments is characterized in that the peptide includes an amino acid sequence selected from the group consisting of SEQ ID NOS: 20, 22, 23, 27, 33, 35, 37, 38, 40, 41, 42, and 44.

In the composition according to any one of the previous specific embodiments, the composition is characterized by exhibiting one or more of the following characteristics of:
(a) lowering the blood pressure, as compared to an excipient-administered control;
(b) reducing albumin excretion, as compared to an excipient-administered control;
(c) reducing proteinuria, as compared to an excipient-administered control; and
(d) restoring the renal function, as compared to an excipient-administered control.

The composition according to any one of the previous specific embodiments is characterized in that the composition exhibits effects of lowering the blood pressure and/or restoring the renal function in a subject, when administered to the subject.

The composition according to any one of the previous specific embodiments is characterized in that the C-terminus of the glucagon derivative is unmodified or amidated.

The composition according to any one of the previous specific embodiments is characterized in that the glucagon derivative has a ring formed between amino acid residues.

The composition according to any one of the previous specific embodiments is characterized in that the immunoglobulin Fc region is aglycosylated.

The composition according to any one of the previous specific embodiments is characterized in that the immunoglobulin Fc region is selected from the group consisting of: (a) a CH1 domain, a CH2 domain, a CH3 domain, and a CH4 domain; (b) a CH1 domain and a CH2 domain; (c) a CH1 domain and a CH3 domain; (d) a CH2 domain and a CH3 domain; (e) a combination between one, or two or more domains, among a CH1 domain, a CH2 domain, a CH3 domain, and a CH4 domain, and an immunoglobulin hinge region or a part of the hinge region; and (f) a dimer of each domain of a heavy chain constant region and a light chain constant region.

The composition according to any one of the previous specific embodiments is characterized in that the immunoglobulin Fc region is an Fc region in which a region capable of forming a disulfide bond is deleted, a part of the amino acids in the N-terminus of a native Fc is deleted, a methionine residue is added to the N-terminus of a native Fc, a complement binding site is deleted, or an antibody-dependent cell-mediated cytotoxicity (ADCC) site is deleted.

The composition according to any one of the previous specific embodiments is characterized in that the immunoglobulin Fc region is derived from IgG, IgA, IgD, IgE, or IgM.

The composition according to any one of the previous specific embodiments is characterized in that the immunoglobulin Fc region is a hybrid of domains with different origins derived from immunoglobulins selected from the group consisting of IgG, IgA, IgD, IgE, and IgM.

The composition according to any one of the previous specific embodiments is characterized in that the immunoglobulin Fc region has a dimeric form.

The composition according to any one of the previous specific embodiments is characterized in that the immunoglobulin Fc region is a dimer consisting of two polypeptide chains, wherein one end of L is linked to only one polypeptide chain of the two polypeptide chains.

The composition according to any one of the previous specific embodiments is characterized in that the immunoglobulin Fc region is an IgG4 Fc region.

The composition according to any one of the previous specific embodiments is characterized in that the immunoglobulin Fc region is an aglycosylated Fc region derived from human IgG4.

The composition according to any one of the previous specific embodiments is characterized in that the immunoglobulin Fc region has a structure in which two polypeptide chains are linked through a disulfide bond, wherein two polypeptide chains are linked through only a nitrogen atom of one of the two chains.

The composition according to any one of the previous specific embodiments is characterized in that the immunoglobulin Fc region includes a monomer having an amino acid sequence of SEQ ID NO: 67.

The composition according to any one of the previous specific embodiments is characterized in that the immunoglobulin Fc region is a homodimer of monomers of the amino acid sequence of SEQ ID NO: 67.

The composition according to any one of the previous specific embodiments is characterized in that the immunoglobulin Fc region is linked through a nitrogen atom of the N-terminal proline.

The composition according to any one of the previous specific embodiments is characterized in that, in the conjugate, L is linked to F and X by covalent linkages formed by reacting one end of L with an amine group or a thiol group of F and by reacting the other end of L with an amine group or a thiol group of X, respectively.

The composition according to any one of the previous specific embodiments is characterized in that L is polyethylene glycol.

The composition according to any one of the previous specific embodiments is characterized in that the formula weight of a moiety of the ethylene glycol repeating units in L is in the range of 1 kDa to 100 kDa.

The composition according to any one of the previous specific embodiments is characterized in that the ethylene glycol repeating units are [OCH₂CH₂]ₙ, wherein n is a natural number and is determined such that an average molecular weight, for example, a number average molecular weight of a moiety of [OCH₂CH₂]ₙ in the conjugate is 1 kDa to 100 kDa.

The composition according to any one of the previous specific embodiments is characterized in that one molecule of X is covalently linked to one Fc region chain of the dimeric immunoglobulin Fc region via the linker containing the ethylene glycol repeating units.

The composition according to any one of the previous specific embodiments is characterized in that one end of the linker is linked to only one Fc region chain of the two Fc region chains of the dimeric immunoglobulin Fc region.

The composition according to any one of the previous specific embodiments is characterized in that the composition further includes a GLP-1 receptor agonist (glucagon-like peptide 1 receptor agonist).

The composition according to any one of the previous specific embodiments is characterized in that the GLP-1 receptor agonist is selected from the group consisting of GLP-1, exendin-3, exendin-4, agonists thereof, derivatives thereof, fragments thereof, variants thereof, and combinations thereof.

The composition according to any one of the previous specific embodiments is characterized in that the GLP-1 receptor agonist is a GLP-1 receptor agonist derivative in which the N-terminal histidine residue is substituted with a substance selected from the group consisting of des-amino-histidyl, dimethyl-histidyl, beta-hydroxy imidazopropionyl, 4-imidazoacetyl, and *beta*-carboxy imidazopropionyl.

The composition according to any one of the previous specific embodiments is characterized in that the GLP-1 receptor agonist is selected from the group consisting of a native exendin-4, an exendin-4 derivative in which the N-terminal amine group of exendin-4 is deleted, an exendin-4 derivative in which the N-terminal amine group of exendin-4 is substituted with a hydroxyl group, an exendin-4 derivative in which the N-terminal amine group of exendin-4 is modified with a dimethyl group, and an exendin-4 derivative in which the *alpha*-carbon of a first amino acid (histidine) of exendin-4 is deleted.

The composition according to any one of the previous specific embodiments is characterized in that the GLP-1 receptor agonist is an imidazo-acetyl exendin-4 (CA exendin-4).

The composition according to any one of the previous specific embodiments is characterized in that the GLP-1 receptor agonist is in the form of a long-acting conjugate in which the GLP-1 receptor agonist is linked to an immunoglobulin Fc region.

The composition according to any one of the previous specific embodiments is characterized in that the GLP-1 receptor agonist is an imidazo-acetyl exendin-4 (CA exendin-4) linked to an immunoglobulin Fc region via a linker containing ethylene glycol repeating units.

Another aspect of the present disclosure provides a method of preventing or treating chronic renal disease, the method including the step of administering, to a subject, the glucagon derivative or conjugate thereof; or the composition including the same.

Still another aspect of the present disclosure provides a method of preventing or treating chronic renal disease, the method including the step of administering, to a subject, the glucagon derivative or conjugate thereof, and the GLP-1 receptor agonist or conjugate thereof; or the composition including the same.

Still another aspect of the present disclosure provides use of the glucagon derivative or conjugate thereof, or the composition including the same in the prevention or treatment of chronic renal disease.

Still another aspect of the present disclosure provides use of the glucagon derivative or conjugate thereof, and the GLP-1 receptor agonist or conjugate thereof; or the composition including the same in the prevention or treatment of chronic renal disease.

Still another aspect of the present disclosure provides use of the glucagon derivative or conjugate thereof, or the composition including the same in the preparation of a medicament for use in the prevention or treatment of chronic renal disease.

Still another aspect of the present disclosure provides use of the glucagon derivative or conjugate thereof, and the GLP-1 receptor agonist or conjugate thereof; or the composition including the same in the preparation of a medicament for use in the prevention or treatment of chronic renal disease.

### [Mode for Invention]

Hereinafter, the present disclosure will be described in more detail.

Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

Throughout the disclosure of the present disclosure, not only the conventional 1-letter codes and 3-letter codes for amino acids present in nature but also the 3-letter codes, such as α-aminoisobutyric acid (Aib), *N*-methylglycine (Sar), *alpha-*methyl-glutamic acid (α-methyl-glutamic acid) generally used for other amino acids, are used. Further, the amino acids mentioned in abbreviation in the present disclosure are described according to the IUPAC-IUB Nomenclature.

| | | | |
|---|---|---|---|
| alanine | Ala, A | arginine | Arg, R |
| asparagine | Asn, N | aspartic acid | Asp, D |
| cysteine | Cys, C | glutamic acid | Glu, E |
| glutamine | Gln, Q | glycine | Gly, G |
| histidine | His, H | isoleucine | Ile, I |
| leucine | Leu, L | lysine | Lys, K |
| methionine | Met, M | phenylalanine | Phe, F |
| proline | Pro, P | serine | Ser, S |
| threonine | Thr, T | tryptophan | Trp, W |
| tyrosine | Tyr, Y | valine | Val, V |

In the present disclosure, "Aib" may be used interchangeably with "2-aminoisobutyric acid" or "aminoisobutyric acid", and 2-aminoisobutyric acid and aminoisobutyric acid may be used interchangeably with each other.

An aspect of the present disclosure provides a composition including a glucagon derivative or a conjugate thereof. Specifically, the composition is a pharmaceutical composition for preventing or treating chronic renal disease (chronic kidney disease), the pharmaceutical composition including the glucagon derivative or conjugate thereof.

In one embodiment, the pharmaceutical composition for preventing or treating chronic renal disease may be a pharmaceutical composition including a pharmaceutically acceptable excipient and the glucagon derivative or conjugate thereof in a pharmaceutically effective amount.

The composition including the glucagon derivative or conjugate thereof as an active ingredient according to the present disclosure may have effects of preventing or treating chronic renal disease by lowering the blood pressure and restoring renal function through actions on glucagon receptors.

The composition of the present disclosure may exhibit the effects of preventing or treating chronic renal disease.

As used herein, the "chronic renal disease (chronic kidney disease, CKD)" may be used interchangeably with chronic kidney disease, and is an independent disease defined when structural or functional abnormalities of the kidney are present for 3 months or more or a glomerular filtration rate decreases to 60 mL/min/1.73 m² or less for 3 months or more regardless of kidney damage. Chronic renal disease is clinically applied by dividing it into five stages according to its progression. When kidney damage is caused by high blood glucose or high blood pressure is maintained for a long time, the risk of developing chronic renal disease increases. In addition, glomerulonephritis, polycystic kidney disease, autoimmune diseases (*e.g.,* lupus), *etc.* are known to cause chronic renal disease.

Patients with chronic renal disease show proteinuria, in which proteins are excreted in the urine due to a decrease in the glomerular filtration rate, or a decline in the kidney function. In particular, hypertension is a cause of chronic renal disease, but it is also understood as a complication of chronic renal disease. In many cases, patients with chronic renal disease are accompanied by glomerular hypertension and proteinuria, and thus it is known that it is necessary to lower the blood pressure and to reduce proteinuria for the treatment of chronic renal disease.

The chronic renal disease may include any one or more disease selected from the group consisting of nephrotic syndrome, renal tubular dysfunction, renal hypertension, uremia, renal failure, and chronic renal failure, but is not limited thereto.

The glucagon derivative or conjugate thereof, or the composition including the same according to the present disclosure may have the effects of lowering the blood pressure and/or restoring renal function.

The lowering of the blood pressure may refer to lowering of the systolic blood pressure (SBP) and the diastolic blood pressure (DBP), but is not limited thereto.

The restoring of renal function may refer to restoring of the intrinsic function of the kidney to filter out waste products in the blood, which may be measured by various methods. As a non-limiting example of the measurement method, albumin excretion, glomerular filtration rate, proteinuria, creatine concentration, *etc.* may be measured, but is not limited thereto.

As used herein, proteinuria refers to a condition in which higher-than-normal levels of proteins (*e.g.,* albumin, globulin, *etc.*) are contained in the urine of chronic renal disease patients with a low glomerular filtration rate. Albumin is a protein that accounts for about 40% of the proteins in urine, and the level of proteinuria may be examined by measuring the albumin level in urine. The glucagon derivative or conjugate thereof, or the composition including the same according to the present disclosure may reduce the amount of proteins (*e.g.,* albumin) excreted from the body by restoring the renal function, and therefore, in the present disclosure, the reduction of proteinuria may be used in the same sense as restoration of renal function.

The glucagon derivative or conjugate thereof, or the composition including the same according to the present disclosure may exhibit the effect of lowering the blood pressure in a subject when administered to the subject. Further, when the glucagon derivative or conjugate thereof, or the composition including the same is administered to a subject, it may restore the subject's renal function. The lowering of blood pressure and the restoring of renal function may be each independently caused or may be caused at the same time by the glucagon derivative or conjugate thereof, or the composition including the same according to the present disclosure.

As used herein, the "subject" refers to mammals including humans, mice, and livestock having renal damage, renal dysfunction, and/or chronic renal disease, and is not particularly limited, as long as beneficial effects may be obtained by lowering the blood pressure and/or by restoring the renal function.

Spontaneously hypertensive rat (SHR) model used in exemplary embodiments of the present disclosure is known as a chronic renal disease-related model, in which renal damage is induced by congenital hypertension, and is used to investigate the efficacy of antihypertensive drugs. In exemplary embodiments of the present disclosure, the effects of lowering the blood pressure and restoring the renal function by the peptide glucagon derivative according to the present disclosure or the long-acting conjugate thereof were confirmed in the above model, indicating its usefulness in the prevention or treatment of chronic renal disease.

The glucagon derivative or conjugate thereof, or the composition including the same according to the present disclosure may exhibit one or more of the following characteristics in a subject to which it is administered, but is not limited thereto:
(a) lowering the blood pressure, as compared to an excipient-administered control;
(b) reducing albumin excretion, as compared to an excipient-administered control;
(c) reducing proteinuria, as compared to an excipient-administered control; and
(d) restoring the renal function, as compared to an excipient-administered control.

The glucagon derivative or conjugate thereof, or the composition including the same according to the present disclosure may have the effects of preventing or treating chronic renal disease by having the above characteristics.

As used herein, "glucagon" is a type of hormone secreted by alpha cells of the pancreas, and functions to promote the decomposition of glycogen and to raise the concentration of glucose, thereby increasing blood glucose levels in the body. Native glucagon may have the following amino acid sequence:

His–Ser–Gln–Gly–Thr–Phe–Thr–Ser–Asp–Tyr–Ser–Lys–Tyr–Leu–Asp–Ser–
Arg–Arg–Ala–Gln–Asp–Phe–Val–Gln–Trp–Leu–Met–Asn–Thr (SEQ ID NO: 1)

The glucagon derivative of the present disclosure includes a peptide having one or more differences in the amino acid sequence compared to native glucagon, a peptide in which the sequence of native glucagon is modified through modification, and a native glucagon mimetic that is able to activate glucagon receptors like native glucagon. Such a derivative of native glucagon may have the effects of lowering the blood pressure and/or restoring the renal function in the body.

Further, the glucagon derivative may be a non-naturally occurring glucagon derivative.

As used herein, the glucagon derivative refers to a peptide capable of acting on glucagon receptors at a significant level, as compared to native glucagon. In the present disclosure, the glucagon derivative may be used interchangeably with "peptide having activity for glucagon receptors", "glucagon analog", or "peptide".

The glucagon derivative of the present disclosure is a peptide having activity for glucagon receptors, wherein the activity for receptors may be exemplified by *in vitro* activity for receptors of 0.1% or more, 0.2% or more, 1% or more, 2% or more, 3 % or more, 4% or more, 5% or more, 6% or more, 7% or more, 8% or more, 9% or more, 10 % or more, 20% or more, 30% or more, 40% or more, 50 % or more, 60% or more, 70% or more, 80% or more, 90 % or more, 100% or more, or 200% or more, as compared to the native, but is not limited thereto.

Specifically, the glucagon derivative of the present disclosure may exhibit *in vitro* activity for glucagon receptors of about 0.001 % or more, about 0.01 % or more, about 0.1% or more, 0.2% or more, about 1% or more, about 2% or more, about 3% or more, about 4% or more, about 5% or more, about 6% or more, about 7% or more, about 8% or more, about 9% or more, about 10% or more, about 20% or more, about 30% or more, about 40% or more, about 50% or more, about 60% or more, about 70% or more, about 80% or more, about 90% or more, about 100%, about 200% or more, as compared to the native ligand (native glucagon) for the corresponding receptors, but significantly increased ranges are included without limitation. Specifically, the peptide having activity for glucagon receptors of the present disclosure may be those having relative activity for glucagon receptors of 5% or more, 10% or more, 20% or more, or 30% or more, as compared to native glucagon, but is not limited thereto.

As used herein, the term "about" refers to a range including all of ±0.5, ±0.4, ±0.3, ±0.2, ±0.1, *etc.,* and it includes all of the values equivalent to those which come immediately after the term "about" or those in a similar range, but is not limited thereto.

The method of measuring the *in vitro* activity of the peptide may employ a variety of methods known in the art (*e.g.,* WO 2016/108586, WO2017/003191, WO 2018/004283), but is not particularly limited thereto.

Regarding the objects of the present disclosure, the glucagon derivative may include a glucagon receptor agonist, or an analog, in which a variation selected from the group consisting of substitution, addition, deletion, modification, and combinations thereof occurs in one or more amino acids in the native glucagon, or a fragment.

As used herein, the "glucagon receptor agonist" has the same meaning as "agent" or "effector", and refers to a substance that binds to glucagon receptors and actually functions like glucagon *in vivo.*

As used herein, the "glucagon analog" refers to a non-native glucagon different from the native glucagon. Such a glucagon analog includes an analog which is modified by addition, deletion, or substitution of a part of amino acids of the native glucagon.

As used herein, the "fragment" refers to a form in which one or more amino acids are deleted from the amino terminal or carboxy terminal of native glucagon or glucagon derivative, while having the effect of preventing or treating chronic renal disease in the body.

The glucagon derivative of the present disclosure may be prepared by modifying some amino acids in native glucagon through any one method of substitution, addition, deletion and modification, or a combination thereof. The substitution of amino acids may generally occur on the basis of similarity in polarity, charge, solubility, hydrophobicity, hydrophilicity, and/or amphipathic nature of the residues, and conservative substitutions with amino acids having similar properties may be expected to exhibit the same or similar activity.

Examples of the glucagon derivatives prepared by a combination of these methods include a peptide having the function of activating glucagon receptors, in which one or more of the amino acid sequence are different from those of native glucagon, and the N-terminal amino acid residue is deaminated, but is not limited thereto, and the derivatives of native glucagon which are applied to the present disclosure may be prepared by a combination of various methods of preparing the derivatives.

Further, the present disclosure includes all of modifications using L-type or D-type amino acids, and/or non-native type amino acids for peptide preparation; and/or modification of native sequence, for example, modification of a side-chain functional group, an intramolecular covalent bonding, e.g., ring formation between side chains, methylation, acylation, ubiquitination, phosphorylation, aminohexanation, biotinylation, *etc.* Further, the modification may also include substitutions into non-native compounds.

Further, the modification may also include all those where one or more amino acids are added to the amino and/or carboxy terminal.

As the amino acids to be substituted or added, not only the 20 amino acids commonly found in human proteins, but also atypical or non-naturally occurring amino acids may be used. Commercial sources of atypical amino acids may include Sigma-Aldrich, ChemPep Inc., and Genzyme Pharmaceuticals. The peptides including these amino acids and typical peptide sequences may be synthesized and purchased from commercial peptide suppliers, e.g., American Peptide Company, Bachem (USA), or Anygen (Korea).

Amino acid derivatives may also be obtained in the same manner, and for example, 4-imidazoacetic acid, *etc.,* may be used.

Further, the glucagon derivative of the present disclosure may be prepared by independently using the above preparation methods or by using a combination thereof.

In one specific embodiment, the glucagon derivative may be a peptide including an amino acid sequence of the following General Formula 1. 
X1-X2-QGTF-X7-SD-X10-S-X12-X13-X14-X15-X16-X17-X18-X19-
X20-X21-F-X23-X24-W-L-X27-X28-X29-X30 (General Formula 1, SEQ ID
NO: 46)
wherein X1 is tyrosine;
X2 is *alpha*-methyl-glutamic acid (α-methyl-glutamic acid), aminoisobutyric acid (Aib), D-alanine, glycine, *N*-methylglycine (Sar), serine, or D-serine;
X7 is threonine, valine, or cysteine;
X10 is tyrosine or cysteine;
X12 is lysine or cysteine;
X13 is tyrosine or cysteine;
X14 is leucine or cysteine;
X15 is aspartic acid, glutamic acid, or cysteine;
X16 is glutamic acid, aspartic acid, serine, *alpha*-methyl-glutamic acid, or cysteine, or is absent;
X17 is aspartic acid, glutamine, glutamic acid, lysine, arginine, serine, cysteine, or valine, or is absent;
X18 is alanine, aspartic acid, glutamic acid, arginine, valine, or cysteine, or is absent;
X19 is alanine, arginine, serine, valine, or cysteine, or is absent;
X20 is lysine, histidine, glutamine, aspartic acid, arginine, *alpha*-methyl-glutamic acid, or cysteine, or is absent;
X21 is aspartic acid, glutamic acid, leucine, valine, or cysteine, or is absent;
X23 is isoleucine, valine, or arginine, or is absent;
X24 is valine, arginine, alanine, cysteine, glutamic acid, lysine, glutamine, alpha-methyl-glutamic acid, or leucine, or is absent;
X27 is isoleucine, valine, alanine, lysine, methionine, glutamine, or arginine, or is absent;
X28 is glutamine, lysine, asparagine, or arginine, or is absent;
X29 is threonine (T);
X30 is cysteine or is absent,
(with the proviso that when the amino acid sequence of General Formula 1 is identical to SEQ ID NO: 1 or SEQ ID NO: 12, it is excluded).

Much more specifically, in General Formula 1,
X2 is aminoisobutyric acid (Aib);
X7 is threonine (T), valine (V), or cysteine (C);
X10 is tyrosine (Y);
X12 is lysine (K);
X13 is tyrosine (Y);
X14 is leucine (L) or cysteine (C);
X15 is aspartic acid (D);
X16 is glutamic acid (E) or serine (S);
X17 is lysine (K), arginine (R), or cysteine (C);
X18 is arginine (R);
X19 is alanine (A) or cysteine (C);
X20 is glutamine (Q) or lysine (K);
X21 is aspartic acid (D) or glutamic acid (E);
X23 is valine (V);
X24 is glutamine (Q);
X27 is methionine (M);
X28 is asparagine (N);
X29 is threonine (T);
X30 is cysteine (C) or is absent.

In another specific embodiment of the present disclosure, the glucagon derivative may be one which includes an amino acid sequence selected from the group consisting of SEQ ID NOS: 2 to 45, and specifically, one which (essentially) consists of an amino acid sequence selected from the group consisting of SEQ ID NOS: 2 to 45, but is not limited thereto. Specifically, the glucagon derivative may be one which includes an amino acid sequence selected from the group consisting of SEQ ID NOS: 2 to 11, 13 to 45, and specifically, one which (essentially) consists of an amino acid sequence selected from the group consisting of SEQ ID NOS: 2 to 11, 13 to 45, but is not limited thereto.

In still another specific embodiment, the glucagon derivative may be one which includes or (essentially) consists of an amino acid sequence selected from the group consisting of SEQ ID NOS: 7 to 11, and 13 to 25, 27, 29, 31, 33, 35 to 45, but is not limited thereto.

Alternatively, the glucagon derivative may be one which includes or (essentially) consists of an amino acid sequence selected from the group consisting of SEQ ID NOS: 20, 22, 23, 27, 33, 35, 37, 38, 40, 41, 42, and 44, but is not limited thereto.

Although described as "consisting of a particular SEQ ID NO" in the present disclosure, such expression does not exclude a mutation that may occur by addition of a meaningless sequence upstream or downstream of the amino acid sequence of the corresponding SEQ ID NO, or a naturally-occurring mutation therein, or a silent mutation therein, as long as the peptide has an activity the same as or corresponding to that of the peptide which consists of an amino acid sequence of the corresponding SEQ ID NO. Even when such a sequence addition or mutation is present, it obviously belongs to the scope of the present disclosure. In other words, when a peptide exhibits at least a predetermined level of homology and exhibits activity for glucagon receptor despite having some sequence differences, such a peptide may fall within the scope of the present disclosure.

For example, the glucagon derivative of the present disclosure may be exemplified by those having at least 60% or more, 65% or more, 70% or more, 75% or more, 80% or more, 85% or more, 90% or more, 91% or more, 92% or more, 93% or more, 94% or more, or 95% or more sequence identity, as compared to the native glucagon, but the glucagon derivative is not limited to specific sequences as long as it has the effects of preventing or treating chronic renal disease. Reference may be made to WO 2016/108586, WO2017/003191, WO 2018/004283 for the glucagon derivative of the present disclosure.

Further, the glucagon derivative of the present disclosure may include a peptide including any one amino acid sequence of SEQ ID NOS: 2 to 45, (essentially) consisting of any one amino acid sequence of SEQ ID NOS: 2 to 45, or having at least 60% or more, 65% or more, 70% or more, 75% or more, 80% or more, 85% or more, 90% or more, 91% or more, 92% or more, 93% or more, 94% or more, or 95% or more sequence identity to any one amino acid sequence of SEQ ID NOS: 2 to 45, and the glucagon derivative is not limited to specific sequences as long as it has the effects of preventing or treating chronic renal disease.

As used herein, the term "homology" or "identity" refers to a degree of relevance between two given amino acid sequences or nucleotide sequences, and the term may be expressed as a percentage.

The terms homology and identity may be often used interchangeably.

Whether or not any two peptide sequences have homology, similarity, or identity may be determined by, for example, a known computer algorithm, such as the "FASTA" program, by using default parameters as in Pearson et al (1988)[Proc. Natl. Acad. Sci. USA 85]: 2444. Alternatively, they may be determined using the Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (including GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, homology, similarity, or identity may be determined using BLAST or ClustalW of the National Center for Biotechnology.

Homology, similarity, or identity of peptides may be determined by comparing sequence information using a GAP computer program, *e.g.,* Needleman et al. (1970), J Mol Biol.48: 443, for example, as disclosed in Smith and Waterman, Adv. Appl. Math (1981) 2:482. Briefly, the GAP program defines similarity as the number of aligned symbols (*i.*e*.,* amino acids) which are similar, divided by the total number of symbols in the shorter of the two sequences. The default parameters for the GAP program may include: (1) a unary comparison matrix (containing a value of 1 for identities and 0 for non-identities) and the weighted comparison matrix (or EDNAFULL (EMBOSS version of NCBI NUC4.4) substitution matrix) of Gribskov et al(1986) Nucl. Acids Res. 14: 6745, as disclosed by Schwartz and Dayhoff, eds., Atlas Of Protein Sequence And Structure, National Biomedical Research Foundation, pp. 353-358 (1979); (2) a penalty of 3.0 for each gap and an additional 0.10 penalty for each symbol in each gap (or gap open penalty 10, gap extension penalty 0.5); and (3) no penalty for end gaps. Therefore, the term "homology" or "identity" used herein represents the relevance between sequences.

The above-described glucagon derivative of the present disclosure may include an intramolecular bridge (*e.g.,* covalent crosslinking or non-covalent crosslinking), and specifically, may be in a form of including a ring between amino acid residues. For example, the glucagon derivative may be in a form in which a ring is formed between the 12^{th} and 16^{th} amino acids or between the 16^{th} and 20^{th} amino acids of the peptide, but is not particularly limited thereto.

Non-limiting examples of the ring may include a lactam crosslinking (or a lactam ring).

Further, the glucagon derivative includes all of those which are modified to include an amino acid capable of forming a ring at the desired site so as to include a ring.

Such a ring may be formed between side chains of amino acids within the glucagon derivative, *e.g.,* in the form of a lactam ring between a side chain of lysine and a side chain of a glutamic acid, but is not particularly limited thereto.

For example, the peptide including the amino acid sequence of General Formula 1 may be one in which each amino acid in each amino acid pair among the amino acid pairs of X10 and X14, X12 and X16, X16 and X20, X17 and X21, X20 and X24, and X24 and X28 in General Formula 1 may be substituted with glutamic acid or lysine, respectively, but is not limited thereto. In Xn (n is a natural number), n refers to the amino acid position from the N-terminus of an amino acid sequence provided.

Further, the peptide including the amino acid sequence of General Formula 1 may be one in which each amino acid in each amino acid pair of X12 and X16 or the amino acid pair of X16 and X20 or the amino acid pair of X17 and X21 is respectively substituted with glutamic acid or lysine, which is capable of forming a ring, but is not limited thereto.

Further, in General Formula 1, the peptide may be one in which a ring (*e.g.,* a lactam ring) is formed between each amino acid in at least one amino acid pair among the amino acid pairs of X10 and X14, X12 and X16, X16 and X20, X17 and X21, X20 and X24, and X24 and X28, but is not limited thereto.

Further, in General Formula 1, X16 may be glutamic acid, X20 may be lysine, and the side chains of X16 and X20 may form a lactam ring, but are not limited thereto.

Meanwhile, the glucagon derivative of the present disclosure may be one in which the N-terminus and/or C-terminus is not modified. However, those variants, where the N-terminus and/or C-terminus thereof is chemically modified or protected by organic groups, or amino acids are added to the end of the glucagon derivative for its protection from proteases *in vivo* while increasing its stability, may also be included in the scope of the glucagon derivative of the present disclosure. In a case where the C-terminus is not modified, the end of the glucagon derivative according to the present disclosure may have a carboxyl group, but is not particularly limited thereto.

In particular, in the case of a chemically-synthesized glucagon derivative, its N- and C-termini are electrically charged, and therefore, in order to remove these charges, acetylation of the N-terminus and/or amidation of the C-terminus may be performed, but is not particularly limited thereto.

Specifically, the N-terminus or the C-terminus of the glucagon derivative of the present disclosure may have an amine group (-NH₂) or a carboxyl group (-COOH), but is not limited thereto.

In the present disclosure, the glucagon derivative may be in the form of a conjugate, in which a biocompatible material moiety capable of increasing *in vivo* half-life thereof is linked, but is not limited thereto.

As used herein, the term "long-acting conjugate" or "conjugate" has a structure in which the glucagon derivative and a biocompatible material are linked to each other, and may exhibit an enhanced efficacy of duration, as compared to the glucagon derivative to which the biocompatible material is not linked. In the long-acting conjugate, the biocompatible material may be covalently linked to the glucagon derivative, but is not particularly limited thereto. In the present disclosure, the glucagon derivative which is one element of the conjugate may be a peptide or a fragment in which substitution, addition, deletion, modification, or a combination thereof occurs in one or more amino acids in the native glucagon sequence, and which does not lose activity by binding of the biocompatible material, but any one may be used as one element of the conjugate of the present disclosure without limitation, as long as it has the effect of preventing or treating chronic renal disease.

For example, the glucagon derivative included in the conjugate may be a peptide including the above-described amino acid sequence of General Formula 1. Alternatively, the glucagon derivative included in the conjugate may be a peptide including any one amino acid sequence of SEQ ID NOS: 2 to 45. Alternatively, the glucagon derivative included in the conjugate may be a peptide including any one amino acid sequence of SEQ ID NOS: 2 to 11, and 13 to 45, but is not limited thereto.

As used herein, the term "biocompatible material" refers to a material which may be linked to the glucagon derivative of the present disclosure which is a physiologically active material, thereby enhancing the efficacy of duration, as compared to the physiologically active material to which the biocompatible material moiety or carrier is not linked. The biocompatible material may be one that is covalently linked to the physiologically active material, but is not particularly limited thereto.

The biocompatible material may be selected from the group consisting of a polymer, fatty acid, cholesterol, albumin and a fragment thereof, an albumin-binding material, a polymer of repeating units of a particular amino acid sequence, an antibody, an antibody fragment, an FcRn-binding material, *in vivo* connective tissue, a nucleotide, fibronectin, transferrin, a saccharide, heparin, and elastin, but is not particularly limited thereto.

For example, the FcRn-binding material may be an immunoglobulin Fc region, more specifically, IgG Fc region, but is not particularly limited thereto.

One or more amino acid side chains within the peptide of the present disclosure may be conjugated to the biocompatible material in order to increase *in vivo* solubility and/or half-life, and/or to increase bioavailability thereof. These modifications may also reduce clearance of therapeutic proteins and peptides.

The above-described biocompatible material may be soluble (amphipathic or hydrophilic) and/or non-toxic and/or pharmaceutically acceptable.

Hereinbelow, the long-acting conjugate of the glucagon derivative which is an active ingredient included in the composition of the present disclosure will be described in more detail.

The long-acting conjugate of the glucagon derivative of the present disclosure refers to a form in which the glucagon derivative and the immunoglobulin Fc region are linked to each other. Specifically, the conjugate may be one in which the immunoglobulin Fc region is covalently linked to the glucagon derivative via a linker, but is not particularly limited thereto.

The long-acting conjugate of the glucagon derivative may exhibit an enhanced efficacy of duration, as compared to the glucagon derivative to which the immunoglobulin Fc region is not linked. In the present disclosure, the conjugate according to Formula 1 of the peptide of General Formula 1 is referred to as the "long-acting conjugate", and may be used interchangeably with the "long-acting conjugate of the glucagon derivative".

Specifically, the glucagon derivative or peptide is in the form of a long-acting conjugate, which may be a conjugate represented by the following Formula 1:

[Formula 1] X-L-F

wherein X represents a peptide (glucagon derivative) including the amino acid sequence of General Formula 1,
L represents a linker containing ethylene glycol repeating units,
F represents an immunoglobulin Fc region, and
- represents covalent linkages between X and L and between L and F, respectively.

The long-acting conjugate of the present disclosure is characterized by including the peptide (glucagon derivative) including the amino acid sequence of General Formula 1 as a part of the conjugate.

The conjugate of the present disclosure may exhibit significant activity for glucagon receptors even in the form of the conjugate, like the glucagon derivative, and therefore, may exhibit the efficacy of preventing or treating chronic renal disease through lowering the blood pressure and restoring the renal function.

Specifically, the conjugate of the present disclosure may have *in vitro* activity for glucagon receptors of about 0.01% or more, 0.1% or more, 0.2% or more, 0.5% or more, 0.7% or more, 1% or more, 2% or more, 3 % or more, 4% or more, 5% or more, 6% or more, 7% or more, 8% or more, 9% or more, 10 % or more, 20% or more, 30% or more, 40% or more, 50 % or more, 60% or more, 70% or more, 80% or more, 90 % or more, 100% or more, as compared to the native glucagon, but is not limited thereto.

Regarding the objects of the present disclosure, the peptide (glucagon derivative) or conjugate thereof may have activity for glucagon receptors of about 0.01 % or more, about 0.1% or more, 1% or more, 2% or more, 3 % or more, 4% or more, 5% or more, 10 % or more, 20% or more, 30% or more, 40% or more, 50 % or more, 60% or more, 70% or more, 80% or more, 90 % or more, 100% or more, 200% or more, as compared to the native glucagon, but is not limited thereto.

The composition of the present disclosure may include (i) the glucagon derivative having activity for glucagon receptors or (ii) the long-acting conjugate of the glucagon derivative, wherein the long-acting conjugate may exhibit the excellent effect of preventing or treating chronic renal disease based on the enhanced *in vivo* efficacy of duration.

In the conjugate, F is a biocompatible material increasing the half-life, specifically, a material capable of increasing the half-life of X, *i.e.*, the glucagon derivative of the present disclosure, and corresponds to one element of the moiety constituting the conjugate of the present disclosure.

F may be linked to X through a covalent chemical bond or a non-covalent chemical bond, and F and X may be linked to each other via L by a covalent chemical bond, a non-covalent chemical bond, or a combination thereof.

More specifically, X and L, and L and F may be linked to each other by a covalent bond, and in this regard, the conjugate is a conjugate in which X, L, and F are linked to each other as in the order of Formula 1 through a covalent bond.

In the present disclosure, F may be an immunoglobulin Fc region.

The "immunoglobulin Fc region" refers to a region including the heavy chain constant region 2 (CH2) and/or the heavy chain constant region 3 (CH3), excluding the heavy chain and light chain variable regions of an immunoglobulin. The immunoglobulin Fc region may be one element that constitutes a moiety of the conjugate of the present disclosure. Specifically, it corresponds to F in Formula 1.

As used herein, the Fc region includes not only the native sequence obtained by papain digestion of immunoglobulins, but also derivatives thereof, for example, variants in which one or more amino acid residues in the native sequence are modified by deletion, insertion, non-conservative or conservative substitution, or a combination thereof and are thus different from those of the native form. The derivatives, substituent, and variants are required to retain FcRn-binding ability. In the present disclosure, F may be a human immunoglobulin region, but is not limited thereto. F has a structure in which two polypeptide chains are linked by a disulfide bond, wherein the two polypeptide chains are linked through only a nitrogen atom of one chain of the two chains, but is not limited thereto. The linking through a nitrogen atom may be linking to the epsilon amino atoms of lysine or the N-terminal amino group through reductive amination.

The reductive amination refers to a reaction in which an amine group or an amino group of one reactant reacts with an aldehyde (*i.e.,* a functional group capable of reductive amination) of another reactant to form an amine, and then an amine linkage is formed by reduction, and reductive amination is an organic synthetic reaction widely known in the art.

In one specific embodiment, F may be linked through a nitrogen atom of the N-terminal proline thereof, but is not limited thereto.

The immunoglobulin Fc region may be one element constituting a moiety of the conjugate of Formula 1 of the present disclosure, and specifically, may correspond to F in Formula 1.

This immunoglobulin Fc region may include a hinge region in a heavy chain constant region, but is not limited thereto.

As used herein, the term "hinge sequence" refers to a site which is located on a heavy chain and forms a dimer of the immunoglobulin Fc region through an inter-disulfide bond.

In the present disclosure, the hinge sequence may be mutated to have only one cysteine residue by deletion of a part in the hinge sequence having the following amino acid sequence, but is not limited thereto:
Glu-Ser-Lys-Tyr-Gly-Pro-Pro-Cys-Pro-Ser-Cys-Pro (SEQ ID NO: 47).

The hinge sequence may include only one cysteine residue by deletion of the 8^{th} or 11^{th} cysteine residues in the hinge sequence of SEQ ID NO: 47. The hinge sequence of the present disclosure may consist of 3 to 12 amino acids including only one cysteine residue, but is not limited thereto. More specifically, the hinge sequence of the present disclosure may have the following sequences:
Glu-Ser-Lys-Tyr-Gly-Pro-Pro-Pro-Ser-Cys-Pro (SEQ ID NO: 48),
Glu-Ser-Lys-Tyr-Gly-Pro-Pro-Cys-Pro-Ser-Pro (SEQ ID NO: 49),
Glu-Ser-Lys-Tyr-Gly-Pro-Pro-Cys-Pro-Ser (SEQ ID NO: 50),
Glu-Ser-Lys-Tyr-Gly-Pro-Pro-Cys-Pro-Pro (SEQ ID NO: 51),
Lys-Tyr-Gly-Pro-Pro-Cys-Pro-Ser (SEQ ID NO: 52),
Glu-Ser-Lys-Tyr-Gly-Pro-Pro-Cys (SEQ ID NO: 53),
Glu-Lys-Tyr-Gly-Pro-Pro-Cys (SEQ ID NO: 54),
Glu-Ser-Pro-Ser-Cys-Pro (SEQ ID NO: 55),
Glu-Pro-Ser-Cys-Pro (SEQ ID NO: 56),
Pro-Ser-Cys-Pro (SEQ ID NO: 57),
Glu-Ser-Lys-Tyr-Gly-Pro-Pro-Ser-Cys-Pro (SEQ ID NO: 58),
Lys-Tyr-Gly-Pro-Pro-Pro-Ser-Cys-Pro (SEQ ID NO: 59),
Glu-Ser-Lys-Tyr-Gly-Pro-Ser-Cys-Pro (SEQ ID NO: 60),
Glu-Ser-Lys-Tyr-Gly-Pro-Pro-Cys (SEQ ID NO: 61),
Lys-Tyr-Gly-Pro-Pro-Cys-Pro (SEQ ID NO: 62),
Glu-Ser-Lys-Pro-Ser-Cys-Pro (SEQ ID NO: 63),
Glu-Ser-Pro-Ser-Cys-Pro (SEQ ID NO: 64),
Glu-Pro-Ser-Cys (SEQ ID NO: 65),
Ser-Cys-Pro (SEQ ID NO: 66).

More Specifically, the hinge sequence may include an amino acid sequence of SEQ ID NO: 57 (Pro-Ser-Cys-Pro) or SEQ ID NO: 66 (Ser-Cys-Pro), but is not limited thereto.

The immunoglobulin Fc region of the present disclosure may be in the form, in which two molecules of the immunoglobulin Fc chain form a dimer due to the presence of the hinge sequence, and the conjugate of Formula 1 of the present disclosure may be in the form, in which one end of the linker is linked to one chain of the dimeric immunoglobulin Fc region, but is not limited thereto.

As used herein, the term "N-terminus" refers to an amino terminus of a protein or polypeptide, and may include the outermost end of the amino terminus, or 1, 2, 3, 4, 5, 6, 7, 8, 9, or 10 or more amino acids from the outermost end. In the immunoglobulin Fc region of the present disclosure, the hinge sequence may be included in the N-terminus thereof, but is not limited thereto.

Further, the immunoglobulin Fc region of the present disclosure may be an extended Fc region including a part or the entirety of heavy chain constant region 1 (CH1) and/or light chain constant region 1 (CL1), excluding only heavy chain and light chain variable regions of an immunoglobulin, as long as the immunoglobulin Fc region has a substantially equivalent or improved effect, as compared to the native form. Further, the immunoglobulin Fc region of the present disclosure may be a region having a deletion of a considerably long partial amino acid sequence corresponding to CH2 and/or CH3.

For example, the immunoglobulin Fc region of the present disclosure may be 1) a CH1 domain, a CH2 domain, a CH3 domain, and a CH4 domain, 2) a CH1 domain and a CH2 domain, 3) a CH1 domain and a CH3 domain, 4) a CH2 domain and a CH3 domain, 5) a combination of one, or two or more domains among a CH1 domain, a CH2 domain, a CH3 domain, and a CH4 domain and an immunoglobulin hinge region (or a part of the hinge region), and 6) a dimer of each domain of a heavy chain constant region and a light chain constant region, but is not limited thereto, but is not limited thereto.

In the present disclosure, the immunoglobulin Fc region may be a dimer or multimer consisting of single-chain immunoglobulins consisting of domains of the same origin, but is not limited thereto.

Further, the immunoglobulin Fc region F is a dimer consisting of two polypeptide chains, wherein the dimeric Fc region F and X may be covalently linked to each other via one identical linker L containing ethylene glycol repeating units. In a specific embodiment, X is covalently linked to only one polypeptide chain of the two polypeptide chains of the dimeric Fc region F via the linker L. In a more specific embodiment, only one molecule of X is covalently linked via L to one polypeptide chain, to which X is linked, of the two polypeptide chains of the dimeric Fc region F. In a most specific embodiment, F is a homodimer.

In another specific embodiment, the immunoglobulin Fc region F is a dimer consisting of two polypeptide chains, and one end of L is linked to only one polypeptide chain of the two polypeptide chains, but is not limited thereto.

In another embodiment of the long-acting conjugate of the present disclosure, it is also possible to symmetrically link two molecules of X to one Fc region in a dimeric form. In this regard, the immunoglobulin Fc and X may be linked to each other via a non-peptide linker, but is not limited to the above-described embodiments.

Further, the immunoglobulin Fc region of the present disclosure includes not only the native amino acid sequence as well as a sequence derivative thereof. The amino acid sequence derivative refers to an amino acid sequence which is different from the native amino acid sequence by deletion, insertion, or non-conservative or conservative substitution of one or more amino acid residues, or a combination thereof.

For example, the amino acid residues at positions 214 to 238, 297 to 299, 318 to 322, or 327 to 331, which are known to be important for linkage in IgG Fc, may be used as appropriate sites for variation.

In addition, various types of derivatives are possible, for example, by removing a region capable of forming a disulfide bond, deleting some amino acid residues at the N-terminus of native Fc, or adding a methionine residue at the N-terminus of native Fc. In addition, in order to remove effector functions, a complement binding site, for example, a C1q binding site, may be removed, and an antibody-dependent cell-mediated cytotoxicity (ADCC) site may be removed. Techniques for preparing such sequence derivatives of the immunoglobulin Fc region are disclosed in WO 97/34631 and WO 96/32478, *etc.*

Amino acid exchanges in a protein or peptide that do not alter the entire activity of a molecule are well known in the art (H. Neurath, R. L. Hill, The Proteins, Academic Press, New York, 1979). The most commonly occurring exchanges are exchanges between amino acid residues Ala/Ser, Val/Ile, Asp/Glu, Thr/Ser, Ala/Gly, Ala/Thr, Ser/Asn, Ala/Val, Ser/Gly, Thy/Phe, Ala/Pro, Lys/Arg, Asp/Asn, Leu/Ile, Leu/Val, Ala/Glu, Asp/Gly. In some cases, amino acids may be modified by phosphorylation, sulfation, acrylation, glycosylation, methylation, farnesylation, acetylation, amidation, *etc.*

The above-described Fc derivatives exhibit a biological activity equivalent to that of the Fc region of the present disclosure, and may be obtained by improving the structural stability of the Fc region against heat, pH, *etc.*

Such an Fc region may be obtained from a native form isolated from living bodies of humans or animals, such as cows, goats, pigs, mice, rabbits, hamsters, rats, and guinea pigs, or may be a recombinant form obtained from transformed animal cells or microorganisms, or derivatives thereof. Here, a method of obtaining from a native form is a method in which the whole immunoglobulin is isolated from a living body of a human or animal and then treated with a protease. The native form may be digested into Fab and Fc when treated with papain and digested into pF'c and F(ab)₂ when treated with pepsin. These may be separated into Fc or pF'c by size-exclusion chromatography, *etc.* In a more specific embodiment, the immunoglobulin Fc region is a recombinant immunoglobulin Fc region obtained from a microorganism.

In addition, the immunoglobulin Fc region may have native glycans, increased glycans compared to the natural type, decreased glycans compared to the natural type, or may be in a deglycosylated form. The increase, decrease, or removal of the glycans of the immunoglobulin Fc may be achieved by common methods such as a chemical method, an enzymatic method, and a genetic engineering method using a microorganism. Here, the immunoglobulin Fc region obtained by removal of glycans from the Fc region shows a significant decrease in binding affinity to the complement (C1q) and a decrease or loss in antibody-dependent cytotoxicity or complement-dependent cytotoxicity, and thus it does not induce unnecessary immune responses *in vivo.* In this regard, a deglycosylated or aglycosylated immunoglobulin Fc region may be a more suitable form to meet the original object as a drug carrier.

As used herein, the term "deglycosylation" refers to enzymatically removing sugar moieties from an Fc region, and the term "aglycosylation" refers to an unglycosylated Fc region produced in prokaryotes, in a more specific embodiment, *E. coli.*

Meanwhile, the immunoglobulin Fc region may be derived from humans or other animals including cows, goats, pigs, mice, rabbits, hamsters, rats, and guinea pigs. In a more specific embodiment, it is derived from humans.

In addition, the immunoglobulin Fc region may be an Fc region derived from IgG, IgA, IgD, IgE, IgM, or a combination or hybrid thereof. In a more specific embodiment, it is derived from IgG or IgM, which are the most abundant in human blood, and in an even more specific embodiment, it is derived from IgG, which is known to enhance the half-lives of ligand-binding proteins. In a yet even more specific embodiment, the immunoglobulin Fc region is an IgG4 Fc region, and in the most specific embodiment, the immunoglobulin Fc region is an aglycosylated Fc region derived from human IgG4, but is not limited thereto.

Further, in a specific embodiment, the immunoglobulin Fc fragment is a region of human IgG4 Fc, may be in the form of a homodimer in which two monomers are linked through a disulfide bond (inter-chain form) between cysteines, which are the 3^{rd} amino acid of each monomer. In this regard, each monomer of the homodimer independently has/may have a disulfide bond between cysteines at positions 35 and 95 and a disulfide bond between cysteines at positions 141 and 199, *i.e.,* two intra-disulfide bonds (intra-chain form).

Each monomer may consist of 221 amino acids, and the number of the amino acids forming the homodimer may be a total of 442, but is not limited thereto. Specifically, in the immunoglobulin Fc region, two monomers having the amino acid sequence of SEQ ID NO: 67 (consisting of 221 amino acids) form a homodimer through a disulfide bond between cysteines, which are the amino acid at position 3 of each monomer, wherein the monomers of the homodimer independently form an intra-disulfide bond between the cysteines at positions 35 and 95 and an intra-disulfide bond between the cysteines at positions 141 and 199, respectively, but is not limited thereto.

F in Formula 1 may include a monomer having the amino acid sequence of SEQ ID NO: 67, wherein F may be a homodimer of monomers having the amino acid sequence of SEQ ID NO: 67, but is not limited thereto.

In an embodiment, the immunoglobulin Fc region may be a homodimer including the amino acid sequence of SEQ ID NO: 68 (consisting of 442 amino acids), but is not limited thereto.

In a specific embodiment, the immunoglobulin Fc region and X may not be glycosylated, but are not limited thereto.

Meanwhile, as used herein, the term "combination" means that when a dimer or multimer is formed, a polypeptide encoding the single-chain immunoglobulin Fc region of the same origin forms a bond with a single-chain polypeptide of a different origin. In other words, it is possible to prepare a dimer or multimer from two or more regions selected from the group consisting of IgG Fc, IgA Fc, IgM Fc, IgD Fc, and IgE Fc regions.

As used herein, the term "hybrid" means that a sequence corresponding to two or more immunoglobulin Fc regions of different origins is present in a single-chain immunoglobulin constant region. In the present disclosure, several types of hybrid are possible. In other words, domains consisting of 1 domain to 4 domains selected from the group consisting of CH1, CH2, CH3, and CH4 of IgG Fc, IgM Fc, IgA Fc, IgE Fc, and IgD Fc may be hybridized, and may include a hinge region.

Meanwhile, IgG may also be divided into IgG1, IgG2, IgG3, and IgG4 subclasses, and a combination or hybridization thereof may also be made in the present disclosure. Specifically, IgG may be IgG2 and IgG4 subclasses, and most specifically, an Fc region of IgG4 having little effector function such as complement dependent cytotoxicity (CDC).

Further, the above-described conjugate may have an increase in the duration of efficacy, as compared to native glucagon, or as compared to X which is not modified with F, and such a conjugate is not only in the above-described form, but also in the form encapsulated in biodegradable nanoparticles.

Meanwhile, in Formula 1 above, L is a non-peptide linker, for example, may be a linker containing ethylene glycol repeating units.

As used herein, the "non-peptide linker" includes a biocompatible polymer in which two or more repeating units are linked. The repeating units are linked with each other by any covalent bond instead of a peptide bond. The non-peptide linker may be one element that constitutes a moiety of the conjugate of the present disclosure, and corresponds to L in Formula 1.

As the non-peptide linker applicable in the present disclosure, any polymer that has resistance to *in vivo* protease may be used without limitation. In the present disclosure, the non-peptide linker may be used interchangeably with a non-peptide polymer.

In the present disclosure, the non-peptide linker includes reactive groups at the ends thereof and thus may form a conjugate by reaction with the other elements constituting the conjugate. When a non-peptide linker having reactive functional groups at both ends thereof binds to X and F in Formula 1 through the respective reactive groups to form a conjugate, the non-peptide linker or non-peptide polymer may be named a non-peptide polymer linker moiety or a non-peptide linker moiety.

Although not particularly limited, the non-peptide linker may be a linker containing ethylene glycol repeating units, and for example, may be polyethylene glycol, and derivatives thereof already known in the art and derivatives that may be easily prepared within the level of skill in the art also fall within the scope of the present disclosure.

The repeating units of the non-peptide linker may be ethylene glycol repeating units, and specifically, the non-peptide linker may include, at ends thereof, functional groups for use in the preparation of a conjugate before being configured into the conjugate, while including ethylene glycol repeating units. The long-acting conjugate according to the present disclosure may be in the form in which X and F are linked through the functional groups, but is not limited thereto. In the present disclosure, the non-peptide linker may include two, or three or more functional groups, and each functional group may be the same as or different from each other, but is not limited thereto.

Specifically, the linker may be polyethylene glycol (PEG) represented by Formula 2 below, but is not limited thereto: 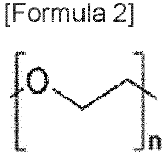 wherein n = 10 to 2400, n = 10 to 480, or n = 50 to 250, but is not limited thereto.

In the long-acting conjugate, the PEG moiety may include not only the - (CH₂CH₂O)ₙ- structure, but also an oxygen atom interposed between a linking element and the -(CH₂CH₂O)ₙ- structure, but is not limited thereto.

In a specific embodiment, the ethylene glycol repeating unit may be represented by, for example, [OCH₂CH₂]ₙ, wherein the value of n is a natural number, and an average molecular weight, for example, a number average molecular weight of the moiety of [OCH₂CH₂]ₙ in the long-acting conjugate may be set to more than 0 kDa to about 100 kDa, but is not limited thereto. As another example, the value of n is a natural number, and an average molecular weight, for example, a number average molecular weight of the moiety of [OCH₂CH₂]ₙ in the long-acting conjugate may be about 1 kDa to about 100 kDa, about 1 kDa to about 80 kDa, about 1 kDa to about 50 kDa, about 1 kDa to about 30 kDa, about 1 kDa to about 25 kDa, about 1 kDa to about 20 kDa, about 1 kDa to about 15 kDa, about 1 kDa to about 13 kDa, about 1 kDa to about 11 kDa, about 1 kDa to about 10 kDa, about 1 kDa to about 8 kDa, about 1 kDa to about 5 kDa, about 1 kDa to about 3.4 kDa, about 3 kDa to about 30 kDa, about 3 kDa to about 27 kDa, about 3 kDa to about 25 kDa, about 3 kDa to about 22 kDa, about 3 kDa to about 20 kDa, about 3 kDa to about 18 kDa, about 3 kDa to about 16 kDa, about 3 kDa to about 15 kDa, about 3 kDa to about 13 kDa, about 3 kDa to about 11 kDa, about 3 kDa to about 10 kDa, about 3 kDa to about 8 kDa, about 3 kDa to about 5 kDa, about 3 kDa to about 3.4 kDa, about 8 kDa to about 30 kDa, about 8 kDa to about 27 kDa, about 8 kDa to about 25 kDa, about 8 kDa to about 22 kDa, about 8 kDa to about 20 kDa, about 8 kDa to about 18 kDa, about 8 kDa to about 16 kDa, about 8 kDa to about 15 kDa, about 8 kDa to about 13 kDa, about 8 kDa to about 11 kDa, about 8 kDa to about 10 kDa, about 9 kDa to about 15 kDa, about 9 kDa to about 14 kDa, about 9 kDa to about 13 kDa, about 9 kDa to about 12 kDa, about 9 kDa to about 11 kDa, about 9.5 kDa to about 10.5 kDa, or about 10 kDa, but is not limited thereto.

In a specific embodiment, the conjugate may have a structure in which the glucagon derivative (X) and the immunoglobulin Fc region (F) are covalently linked via a linker containing ethylene glycol repeating units, but is not limited thereto.

In another specific embodiment, in the long-acting conjugate, L may be a linker containing ethylene glycol repeating units, and F may be a dimeric immunoglobulin Fc region. More specifically, one molecule of X may be covalently linked to one Fc region of the dimeric immunoglobulin Fc region via the linker containing ethylene glycol repeating units, but is not limited thereto. Further, in still another specific embodiment, one end of the linker containing ethylene glycol repeating units may be linked to only one Fc region chain of the two Fc region chains of the dimeric immunoglobulin Fc region, but is not limited thereto.

The polyethylene glycol is a term encompassing all of the forms of homopolymers of ethylene glycol, PEG copolymers, and monomethyl-substituted PEG polymers (mPEG), but is not particularly limited thereto.

The formula weight of the ethylene glycol repeating units in L may be in the range of 1 kDa to 100 kDa, but is not limited thereto. In addition, in one specific embodiment, the conjugate may be one in which F, specifically the immunoglobulin Fc region, and X, specifically the glucagon derivative, are covalently linked to each other via the non-peptide linker containing reactive groups capable of binding with F and X at both ends thereof.

As the non-peptide linker applicable in the present disclosure, any polymer that has resistance to proteases *in vivo* and contains ethylene glycol units may be used without limitation. The molecular weight of the non-peptide polymer may be in the range of more than 0 kDa to about 100 kDa, or about 1 kDa to about 100 kDa, and specifically, in the range of about 1 kDa to about 20 kDa, or about 1 kDa to about 10 kDa, but is not limited thereto. In addition, the non-peptide linker of the present disclosure, which is linked to the polypeptide corresponding to F, may include not only a single type of polymer but also a combination of different types of polymers.

As used herein, the term "about" refers to a range including all of ±0.5, ±0.4, ±0.3, ±0.2, ±0.1, etc., and it includes all of the values equivalent to those which come immediately after the term "about" or those in a similar range, but is not limited thereto.

Specifically, the non-peptide linker may have reactive groups at both ends while being not bound with F and X, and may be bound with F and X through the reactive groups.

In a specific embodiment, both ends of the linker may be linked to a thiol group, an amino group, or a hydroxyl group of the immunoglobulin Fc region and a thiol group, an amino group, an azide group, or a hydroxyl group of the glucagon derivative (X), respectively, but are not limited thereto.

Specifically, the linker may include, at both ends thereof, reactive groups capable of binding to the immunoglobulin Fc region and the glucagon derivative (X), respectively, specifically, reactive groups capable of binding to a thiol group of cysteine; an amino group located at the N-terminus, lysine, arginine, glutamine, and/or histidine; and/or a hydroxyl group located at the C-terminus in the immunoglobulin Fc region; and a thiol group of cysteine; an amino group of lysine, arginine, glutamine, and/or histidine; an azide group of azido-lysine; and/or a hydroxyl group in the glucagon derivative (X), but is not limited thereto.

More specifically, each of the reactive groups of the linker may be one or more selected from the group consisting of an aldehyde group, a maleimide group, and a succinimide derivative, but is not limited thereto.

In the above, the aldehyde group may be exemplified by a propionaldehyde group or a butyraldehyde group, but is not limited thereto.

In the above, the succinimide derivative may be exemplified by succinimidyl valerate, succinimidyl methylbutanoate, succinimidyl methylpropionate, succinimidyl butanoate, succinimidyl propionate, *N*-hydroxysuccinimide, hydroxy succinimidyl, succinimidyl carboxymethyl, or succinimidyl carbonate, but is not limited thereto.

The linker may be linked to the immunoglobulin Fc region F and the glucagon derivative X via such reactive groups to be converted into a linker moiety.

Further, a final product produced by reductive amination through aldehyde linkage is still more stable than one obtained through amide linkage. The aldehyde reactive group selectively reacts with the N-terminus at low pH while forming a covalent linkage with a lysine residue at high pH, for example, at pH 9.0.

Further, the reactive groups of both ends of the non-peptide linker may be the same as or different from each other, and for example, the non-peptide linker may have aldehyde groups at both ends, or a maleimide group at one end and an aldehyde group, a propionaldehyde group, or a butyraldehyde group at the other end. However, the reactive groups are not particularly limited thereto as long as F, specifically, the immunoglobulin Fc region, and X may be linked to each end of the non-peptide linker.

For example, the non-peptide linker may include a maleimide group as a reactive group at one end and an aldehyde group, a propionaldehyde group, a butyraldehyde group, *etc.* at the other end.

When polyethylene glycol having hydroxyl reactive groups at both ends is used as the non-peptide polymer, the long-acting protein conjugate of the present disclosure may be prepared by activating the hydroxyl groups into various reactive groups through known chemical reactions or by using commercially available polyethylene glycol having modified reactive groups.

In a specific embodiment, the non-peptide polymer may be linked to a cysteine residue of X, more specifically, a -SH group of cysteine, but is not limited thereto.

For example, the non-peptide polymer may be linked to the cysteine residue at position 7, the cysteine residue at position 10, the cysteine residue at position 12, the cysteine residue at position 13, the cysteine residue at position 14, the cysteine residue at position 15, the cysteine residue at position 16, the cysteine residue at position 17, the cysteine residue at position 18, the cysteine residue at position 19, the cysteine residue at position 20, the cysteine residue at position 21, or the cysteine residue at position 30 in the peptide corresponding to X, but is not particularly limited thereto.

Specifically, the reactive group of the non-peptide polymer may be linked to the -SH group of the cysteine residue, and all of those described above are applied to the reactive group. When maleimide-PEG-aldehyde is used, the maleimide group may be linked to the -SH group of X through a thioether linkage, and the aldehyde group may be linked to the -NH₂ group of F, specifically the immunoglobulin Fc, through reductive amination, but is not limited thereto, and this corresponds to one example.

Through such reductive alkylation, the N-terminal amino group of the immunoglobulin Fc fragment is linked to an oxygen atom located at one end of PEG via a linker reactive group having a structure of -CH₂CH₂CH₂- to form a structure, like -PEG-O-CH₂CH₂CH₂NH-immunoglobulin Fc, and through a thioether linkage, a structure in which one end of PEG is linked to a sulfur atom located at cysteine of the peptide of General Formula 1 may be formed. The above-described thioether linkage may contain a structure of

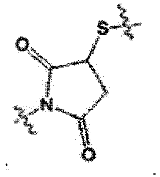

However, the non-peptide polymer is not particularly limited to the above embodiment, and this corresponds to one example.

In another specific embodiment, the non-peptide polymer may be linked to a lysine residue of X, more specifically, the amino group of the lysine, but is not limited thereto.

In the conjugate, the reactive group of the non-peptide polymer may be linked to -NH₂ located at the N-terminus of the immunoglobulin Fc region, and this corresponds to one example.

Further, in the conjugate, the glucagon derivative according to the present disclosure may be linked to a linker having a reactive group through the C-terminus thereof, but this corresponds to one example.

As used herein, the term "C-terminus" refers to a carboxy terminus of the peptide, and regarding the objects of the present disclosure, the term refers to a position that may be linked with a linker. For example, although not limited, the C-terminus may include not only the amino acid residue at the outermost end of the C-terminus but also amino acid residues near the C-terminus, and specifically, the 1^{st} to 20^{th} amino acid residues from the outermost end, but is not limited thereto.

In a specific embodiment, the conjugate of Formula 1 may have a structure of the following Formula 3:

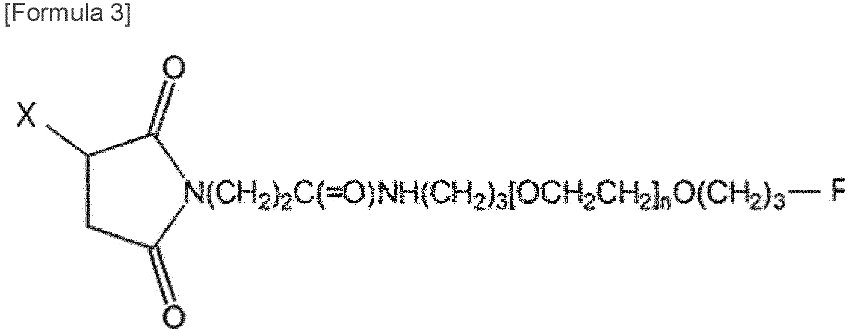

In Formula 3, X is the above-described peptide of General Formula 1;
F is a human immunoglobulin Fc region; and
n may be a natural number, wherein n is the same as explained above.

In a specific embodiment, the long-acting conjugate of Formula 3 may have a structure in which the peptide X of General Formula 1 of SEQ ID NO: 46 and a human immunoglobulin Fc region F are covalently linked via ethylene glycol repeating units, wherein X may be linked to a succinimide ring of Formula 3 and F may be linked to an oxypropylene group of Formula 3.

In Formula 3, the value of n may be determined such that an average molecular weight, for example, a number average molecular weight of a moiety of [OCH₂CH₂]ₙ in the peptide conjugate is 1 kDa to 100 kDa, or 1 kDa to 20 kDa, or 10 kDa, but is not limited thereto.

X of the conjugate may be a peptide having activity for glucagon receptors.

In one embodiment, the region of X linked to the succinimide ring of Formula 3 may be a sulfur atom of the C-terminal cysteine of X.

In Formula 3, F is a human immunoglobulin Fc region. The moiety linked to the oxypropylene group in F is not particularly limited. In one embodiment of the present disclosure, the moiety of F linked to the oxypropylene group may be a N-terminal nitrogen or a nitrogen atom of a residue in F (*e.g.,* epsilon nitrogen of lysine). In one specific embodiment of the present disclosure, the moiety where F is linked to the oxypropylene group of Chemical Formula 3 may be the N-terminal proline of F, but is not limited thereto.

In one specific embodiment, F may be linked through a nitrogen atom in the N-terminal proline thereof, but is not limited thereto.

The type of the glucagon derivative may be exemplified by the types described in WO 2016/108586 and WO 2017/003191, and the entire specification of the above International Publication Patents is incorporated herein by reference. In addition, the method of preparing the long-acting conjugate of the glucagon derivative is described in WO 2017/003191, and it is obvious that the entire specification of the International Publication Patent is also incorporated herein by reference.

Further, the above-described conjugate may have an enhanced efficacy of duration, as compared to X in which F is not modified, and such a conjugate includes not only the above-described form, but also the form encapsulated in biodegradable nanoparticles.

Unless specified otherwise in the present disclosure, the description in the detailed description or claims with respect to "the peptide" according to the present disclosure or the "conjugate", in which such a peptide is covalently linked to a biocompatible material, may be applied to the forms, which include not only include the corresponding peptide or conjugate but also the salts of the corresponding peptide or conjugate (*e.g.,* pharmaceutically acceptable salts thereof), or solvates thereof. Accordingly, even in a case where a "peptide" or "conjugate" is described in the present disclosure, the description may also be equally applied to a particular salt thereof, a particular solvate thereof, and a particular solvate of the particular salt thereof. These salts may be, for example, in a form where any pharmaceutically acceptable salts are used. The kind of the salt is not particularly limited. However, the salt is preferably one that is safe and effective to a subject, *e.g.,* a mammal, but is not particularly limited thereto.

The term "pharmaceutically acceptable" refers to a material which may be effectively used for the desired use within the scope of pharmaco-medical decision without inducing excessive toxicity, irritation, allergic responses, *etc.*

As used herein, the term "pharmaceutically acceptable salt" refers to a salt derived from pharmaceutically acceptable inorganic salts, organic salts, or bases. Examples of the suitable salts may include hydrochloric acid, bromic acid, sulfuric acid, nitric acid, perchloric acid, fumaric acid, maleic acid, phosphoric acid, glycolic acid, lactic acid, salicylic acid, succinic acid, toluene-*p*-sulfonic acid, tartaric acid, acetic acid, citric acid, methanesulfonic acid, formic acid, benzoic acid, malonic acid, naphthalene-2-sulfonic acid, benzenesulfonic acid, *etc.* Examples of the salts derived from suitable bases may include alkali metals such as sodium, potassium, *etc.*; alkali earth metals such as magnesium; ammonium, *etc.*

Further, as used herein, the term "solvate" refers to a complex formed between the peptide, conjugate, or a salt thereof according to the present disclosure and a solvent molecule.

Further, the peptide of the present disclosure may be synthesized by a method well-known in the art, according to its length, *e.g.,* by an automatic peptide synthesizer, and may be produced by a genetic engineering technology.

Specifically, the peptide of the present disclosure may be prepared by a standard synthesis method, a recombinant expression system, or any other method known in the art. Accordingly, the glucagon derivative of the present disclosure may be synthesized by various methods including, for example, the methods described below:
(a) a method of synthesizing a peptide by a solid-phase or liquid-phase method stepwise or by fragment assembly, followed by isolation and purification of the final peptide product; or
(b) a method of expressing a nucleic acid construct encoding a peptide in a host cell and recovering the expression product from the host cell culture; or
(c) a method of performing an *in vitro* cell-free expression of a nucleic acid construct encoding a peptide and recovering the expression product therefrom; or
   a method of obtaining peptide fragments by any combination of the methods (a), (b), and (c), obtaining the peptide by linking the peptide fragments, and then recovering the peptide.

The composition according to the present disclosure may include the glucagon derivative or conjugate thereof, specifically, a pharmaceutically effective amount of the glucagon derivative or conjugate thereof. In addition, the composition according to the present disclosure may further include a pharmaceutically acceptable carrier. The composition of the present disclosure may have use for the prevention or treatment of chronic renal disease, and may have the effects of lowering the blood pressure and/or restoring the renal function, but is not limited thereto.

As used herein, the term "pharmaceutical acceptable" refers to a sufficient amount to show therapeutic effects without causing side effects, and the amount may be easily determined by a person skilled in the art according to factors that are well known in the medical field, including the type of disease, patient's age, body weight, health condition, and sex, sensitivity of a patient to drugs, the route of administration, the method of administration, the number of times of administration, the period of treatment, drugs used in combination or at the same time, and the like.

Regarding the pharmaceutically acceptable carrier, a binder, a lubricant, a disintegrant, an excipient, a solubilizer, a dispersant, a stabilizer, a suspending agent, a coloring agent, a flavoring agent, *etc.* may be used for oral administration; a buffer, a preservative, an analgesic, a solubilizer, an isotonic agent, a stabilizer, *etc.* may be used in combination for injectable preparations; and a base, an excipient, a lubricant, a preservative, *etc.* may be used for topical administration. The formulations of the pharmaceutical composition of the present disclosure may be prepared in various manners by mixing with the pharmaceutically acceptable carriers described above. For example, for oral administration, the pharmaceutical composition may be formulated in the form of a tablet, a troche, a capsule, an elixir, a suspension, a syrup, a wafer, *etc.*; and for injectable preparations, the pharmaceutical composition may be formulated in the form of a unit-dosing ampoule or a multidosing form. In addition, the pharmaceutical composition may also be formulated into a solution, a suspension, a tablet, pills, a capsule, a sustained-release preparation, *etc.*

Meanwhile, examples of a carrier, an excipient, and a diluent suitable for the formulation may include lactose, dextrose, sucrose, sorbitol, mannitol, xylitol, erythritol, maltitol, starch, acacia, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, mineral oils, *etc.* In addition, a filler, an anti-coagulant, a lubricant, a wetting agent, a flavor, an emulsifier, a preservative, *etc.* may be further included.

Further, the composition of the present disclosure may have any one formulation selected from the group consisting of a tablet, a pill, a powder, granules, a capsule, a suspension, a liquid preparation for internal use, an emulsion, a syrup, a sterile aqueous solution, a non-aqueous solvent, a lyophilized preparation, and a suppository.

In addition, the conjugate may be used by mixing with various carriers approved as drugs, such as physiological saline or organic solvents, and for increasing stability or absorptivity, carbohydrates such as glucose, sucrose, or dextran, antioxidants such as ascorbic acid or glutathione, chelating agents, low-molecular-weight proteins, or other stabilizers may be used as medical agents.

The dose and frequency of the composition of the present disclosure are determined according to the type of drug as an active ingredient, along with various factors, such as a disease to be treated, a route of administration, patient's age, sex, and body weight, and severity of a disease. Specifically, the composition of the present disclosure may include a pharmaceutically effective amount of the glucagon derivative or conjugate thereof, but is not limited thereto.

Including a pharmaceutically effective amount of the glucagon derivative or conjugate thereof refers to a level at which desired pharmaceutical activity (*e.g.,* prevention or treatment of chronic renal disease) may be obtained by the glucagon derivative or conjugate thereof, and may also refer to a level at which toxicities or side effects do not occur or occur at an insignificant level or at a pharmaceutically acceptable level in a subject to be administered, but the level is not limited thereto. Such a pharmaceutically effective amount may be determined by comprehensively considering the frequency of administration, patient, formulations, *etc.*

Although not particularly limited, the pharmaceutical composition of the present disclosure may include the ingredient (active ingredient) in an amount of 0.01% (w/v) to 99% (w/v).

The total effective dose of the composition of the present disclosure may be administered to a patient in a single dose, or may be administered in multiple doses according to a fractionated treatment protocol for a long period of time. In the composition of the present disclosure, the content of an active ingredient may vary according to the severity of a disease. Specifically, the preferable total daily dose of the conjugate of the present disclosure may be about 0.0001 mg to 500 mg per 1 kg of body weight of a patient. However, the effective administration dose of the conjugate is determined considering various factors including patient's age, body weight, health condition, sex, severity of a disease, a diet, and an excretion rate, in addition to the route of administration and the frequency of treatment of the pharmaceutical composition. Therefore, considering these, a person skilled in the art may easily determine an appropriate effective dose according to particular uses of the composition of the present disclosure. The pharmaceutical composition according to the present disclosure is not particularly limited to the formation, route of administration, and method of administration, as long as the pharmaceutical composition shows the effects of the present disclosure.

For example, the composition of the present disclosure may be administered once a week, once every 2 weeks, once every 4 weeks, or once a month, but is not limited thereto.

Another specific aspect of the composition according to the present disclosure may further include a GLP-1 receptor agonist (glucagon-like peptide 1 receptor agonist), but is not limited thereto.

Specifically, the composition of the present disclosure may be a pharmaceutical composition for preventing or treating chronic renal disease, the pharmaceutical composition including the glucagon derivative and the GLP-1 receptor agonist, more specifically, a pharmaceutical composition for preventing or treating chronic renal disease, the pharmaceutical composition including the glucagon derivative, the GLP-1 receptor agonist, and a pharmaceutically acceptable excipient, but is not limited thereto.

The pharmaceutical composition of the present disclosure including (i) the glucagon derivative; and (ii) the GLP-1 receptor agonist may be in the form which may be administered
a) in a mixture in which (i) the glucagon derivative; and (ii) the GLP-1 receptor agonist are mixed; or
b) in a form in which (i) the glucagon derivative; and (ii) the GLP-1 receptor agonist are separated, but is not limited thereto.

For example, the glucagon derivative and the GLP-1 receptor agonist are formulated into one preparation, or individually formulated. When the glucagon derivative; and the GLP-1 receptor agonist are separated, the glucagon derivative and the GLP-1 receptor agonist are formulated into individual preparations, and administered simultaneously, individually, sequentially, or in reverse order.

In the present disclosure, combined administration must be understood not only as simultaneous administration, but also as an administration form in which the glucagon derivative and the GLP-1 receptor agonist act together on a subject so that each substance may perform its original function at the same level or higher. Accordingly, as used herein, the term "combination" must be understood to refer to a simultaneous, individual, sequential, or reverse-order administration of the glucagon derivative and the GLP-1 receptor agonist. When the administration is a sequential, reverse-order, or individual administration, the order of administration is not particularly limited, but the interval for the administration of the secondary ingredient must be one which does not lose the advantageous effect of the combined administration.

The glucagon derivative and the GLP-1 receptor agonist of the present disclosure, or the composition including the same may be provided in the form of a kit, but is not limited thereto. As used herein, the term "kit" may include the composition according to the present disclosure for combined administration of the glucagon derivative and the GLP-1 receptor agonist. Specifically, the kit according to the present disclosure may include the glucagon derivative and the GLP-1 receptor agonist which are formulated into one preparation, or individual preparations of the glucagon derivative and the GLP-1 receptor agonist, and may further include substances required for combined administration of two substances, but is not limited thereto.

Meanwhile, the glucagon derivative and the GLP-1 receptor agonist may be in the form of a long-acting conjugate in which an immunoglobulin Fc region is linked, but is not limited thereto.

Glucagon-like peptide-1 (GLP-1) is a hormone secreted from the small intestine and is known to promote intracellular glucose uptake. In the small intestine, glucagon precursors are broken down into three peptides: glucagon, GLP-1, and GLP-2. Here, GLP-1 means GLP-1(1-37), which has no insulin secretion function, and is processed into GLP-1(7-37) form to become active GLP-1(7-37). The GLP-1(7-37) amino acid sequence is shown below:
GLP-1(7-37):
HAEGT FTSDV SSYLE GQAAK EFIAW LVKGR G (SEQ ID NO: 69)

GLP-1 receptor agonist refers to an agonist capable of acting on the GLP-1 receptor, and may include various substances, such as compounds or peptide-type substances, but is not limited thereto.

The GLP-1 receptor agonist may be a substance (e.g., peptide) having *in vitro* activity for GLP-1 receptor at a significant level of 0.1% or more, 1% or more, 2% or more, 3 % or more, 4% or more, 5% or more, 6% or more, 7% or more, 8% or more, 9% or more, 10 % or more, 20% or more, 30% or more, 40% or more, 50 % or more, 60% or more, 70% or more, 80% or more, 90 % or more, 100% or more, as compared to a native ligand (native GLP-1) of the corresponding receptor as well as a native GLP-1, but is not limited thereto.

The method of measuring the *in vitro* activity of the GLP-1 receptor agonist may be achieved by various methods of measuring the *in vitro* activity known in the art.

The GLP-1 receptor agonist according to the present disclosure includes a peptide having one or more differences in the amino acid sequence compared to native GLP-1 while having a significant level of activity for GLP-1 receptor, a peptide in which the sequence of native GLP-1 is modified through modification, or a native GLP-1 mimetic, *etc.,* but is not limited thereto.

Specifically, the GLP-1 receptor agonist of the present disclosure may include those selected from the group consisting of GLP-1, exendin-3, exendin-4, agonists thereof, derivatives thereof, fragments thereof, variants thereof, and combinations thereof, but is not limited thereto.

In the present disclosure, the "agonist" has the same meaning as "agent" or "effector", and refers to a substance that binds to receptors and actually functions like the native ligand *in vivo.*

In the present disclosure, the "derivative" may be in a form of having a significant level of activity for GLP-1 receptor, in which some sequences of native GLP-1 are modified, and some groups on amino acid residues are chemically substituted (e.g., alpha-methylation, alpha-hydroxylation), deleted (e.g., deamination), or modified (e.g., N-methylation), but is not limited thereto.

The GLP-1 receptor agonist of the present disclosure may include a derivative (desamino-histidyl derivative) with removal of the N-terminal amino group, a derivative (*beta*-hydroxy imidazopropionyl-derivative) prepared by substitution of the amino group with a hydroxyl group, a derivative (dimethyl-histidyl derivative) prepared by modification of the amino group with two methyl residues, a derivative (*beta*-carboxy imidazopropionyl derivative) prepared by substitution of the amino-terminal amino group with a carboxyl group, or a derivative (imidazoacetyl derivative) with removal of the positive charge of the amino group, in which the *alpha*-carbon of the amino-terminal histidine residue is removed to remain only the imidazoacetyl group, *etc.,* and other amino-terminal amino group modified-derivatives fall within the scope of the present disclosure.

Specific examples of the GLP-1 receptor agonist of the present disclosure may include derivatives having chemically modified N-terminal amino group or amino acid residue of exendin-4, more specifically, exendin-4 derivative in which the *alpha-*amino group or *alpha*-carbon of a histidine residue, which is the 1^{st} amino acid of the amino terminus of exendin-4, is substituted or removed, and much more specifically, desamino-histidyl-exendin-4 (DA-Exendin-4) with removal of the N-terminal amino group, *beta*-hydroxy imidazopropionyl-exendin-4 (HY-exendin-4) prepared by substitution with a hydroxyl group or a carboxyl group, *beta*-carboxy imidazopropionyl-exendin-4 (CX-exendin-4), dimethyl-histidyl-exendin-4 (DM-exendin-4) prepared by modification with two methyl residues, and imidazoacetyl-exendin-4 (CA-exendin-4) with removal of the *alpha*-carbon of amino-terminal histidine residue, but are not limited thereto.

Non-limiting examples of the GLP-1 receptor agonist of the present disclosure may include a GLP-1 receptor agonist derivative in which the N-terminal histidine residue is substituted with a substance selected from the group consisting of desamino-histidyl, dimethyl-histidyl, *beta*-hydroxy imidazopropionyl, 4-imidazoacetyl, and *beta*-carboxy imidazopropionyl.

As used herein, the "GLP-1 fragment" refers to a form having one or more amino acids added or deleted at the N-terminus or the C-terminus of the native GLP-1, in which the added amino acids are non-naturally occurring amino acids (*e.g.,* D-type amino acid), while having a significant level of activity for GLP-1 receptor.

As used herein, the "GLP-1 variant" refers to a peptide having one or more amino acid sequences different from those of the native GLP-1, while having a significant level of activity for GLP-1 receptor, and may be used interchangeably with "analog".

Specifically, the GLP-1 variant may be modified by any one of substitution, addition, deletion, and modification, or by a combination thereof in a part of the amino acids of the native GLP-1.

Examples of the GLP-1 variant prepared by a combination of these methods include a peptide having the function of activating GLP-1 receptors, in which one or more of the amino acid sequence are different, as compared to that of native GLP-1, and in which the N-terminal amino acid residue is deaminated, but is not limited thereto.

Further, the modification may also include all those where one or more amino acids are added to the amino and/or carboxy terminus of the native GLP-1. As the amino acids to be substituted or added, not only the 20 amino acids commonly found in human proteins, but also atypical or non-naturally occurring amino acids may be used. Commercial sources of atypical amino acids may include Sigma-Aldrich, ChemPep Inc., and Genzyme Pharmaceuticals. The peptides including these amino acids and typical peptide sequences may be synthesized and purchased from commercial peptide suppliers, *e.g.,* American Peptide Company, Bachem (USA), or Anygen (Korea). Amino acid derivatives may also be obtained in the same manner, and for example, 4-imidazoacetic acid, *etc.* may be used.

Further, such modification for the preparation of the derivative or variant includes all of modifications using L-type or D-type amino acids, and/or non-native type amino acids; and/or modification of native sequence, for example, modification of a side-chain functional group, an intramolecular covalent bonding, e.g., ring formation between side chains, methylation, acylation, ubiquitination, phosphorylation, aminohexanation, biotinylation, *etc.* Further, the modification may also include substitutions into non-native compounds.

The exendin-3 and exendin-4 of the present disclosure are GLP-1 receptor agonists consisting of 39 amino acids showing 53% similarity of the amino acid sequence with GLP-1, and amino acid sequences of exendin-3 and exendin-4 are as follows:
Exendin-3:
   HSDGT FTSDL SKQME EEAVR LFIEW LKNGG PSSGA PPPS (SEQ ID NO: 70)
Exendin-4:
   HGEGT FTSDL SKQME EEAVR LFIEW LKNGG PSSGA PPPS (SEQ ID NO: 71)

The exendin agonist refers to a substance which binds to *in vivo* receptor of exendin, regardless of the structure of exendin, and exhibits the same biological activity as exendin, and the exendin derivative may be in a form of showing at least 80% or more homology in the amino acid sequence, as compared to the native exendin, and in which some groups on amino acid residues are chemically substituted (*e.g., alpha*-methylation, *alpha*-hydroxylation), deleted (*e.g.,* deamination), or modified (*e.g.,* N-methylation), but is not limited thereto.

The exendin derivative may include a derivative (desamino-histidyl derivative) with removal of the N-terminal amino group, a derivative (*beta*-hydroxy imidazopropionyl-derivative) prepared by substitution of the amino group with a hydroxyl group, a derivative (dimethyl-histidyl derivative) prepared by modification of the amino group with two methyl residues, a derivative (*beta*-carboxy imidazopropionyl derivative) prepared by substitution of the amino-terminal amino group with a carboxyl group, or a derivative (imidazoacetyl derivative) with removal of the positive charge of the amino group, in which the *alpha*-carbon of the amino-terminal histidine residue is removed to remain only the imidazoacetyl group, *etc.,* and other amino-terminal amino group modified-derivatives fall within the scope of the present disclosure.

Specific examples of the exendin derivative of the present disclosure may include derivatives having chemically modified N-terminal amino group or amino acid residue of exendin-4, more specifically, exendin-4 derivative in which the alpha amino group or *alpha*-carbon of a histidine residue, which is the 1^{st} amino acid of the amino terminus of exendin-4, is substituted or removed, and much more specifically, desamino-histidyl-exendin-4 (DA-Exendin-4) with removal of the N-terminal amino group, *beta*-hydroxy imidazopropionyl-exendin-4 (HY-exendin-4) prepared by substitution with a hydroxyl group or a carboxyl group, *beta*-carboxy imidazopropionyl-exendin-4 (CX-exendin-4), dimethyl-histidyl-exendin-4 (DM-exendin-4) prepared by modification with two methyl residues, and imidazoacetyl-exendin-4 (CA-exendin-4) with removal of *alpha*-carbon of amino-terminal histidine residue, but are not limited thereto.

Non-limiting examples of the exendin derivative of the present disclosure may include an exendin-4 derivative prepared by deleting the N-terminal amine group of exendin-4, an exendin-4 derivative prepared by substituting the N-terminal amine group of exendin-4 with a hydroxyl group, an exendin-4 derivative in which the N-terminal amine group of exendin-4 is modified with a dimethyl group, or an exendin-4 derivative prepared by deleting the *alpha*-carbon of the 1^{st} amino acid (histidine) of exendin-4, but are not limited thereto.

The exendin fragment refers to a form having one or more amino acids added or deleted at the N-terminus or the C-terminus of the native exendin, in which addition of non-naturally occurring amino acids (*e*.*g.,* D-type amino acid) is also possible.

The exendin variant refers to a peptide having one or more amino acid sequences different from those of the native exendin, and specifically, may be modified by any one of substitution, addition, deletion, and modification or by a combination thereof in a part of the amino acids of the native exendin.

Respective methods of preparing the agonist, the derivative, the fragment, and the variant may be used independently or in combination. For example, an insulinotropic peptide, in which one or more of the amino acid sequence are different and the N-terminal amino acid residue is deaminated, is also included.

Specific examples of the GLP-1 receptor agonist of the present disclosure may include [(1*H*-imidazol-4-yl)-acetyl1]GEGTFTSDL SKQMEEEAVR LFIEWLKNGGPSSGAPPPS (SEQ ID NO: 72) (Bachem) or HGEGTFTSDV SSYLEEQAAK EFIAWLVKG (SEQ ID NO: 73) (Bachem), but are not limited thereto. The amino acid sequence is written in the direction from the N-terminus to the C-terminus. Reference may be made to WO 2015-005748 A1 for the GLP-1 receptor agonist of the present disclosure.

In the present disclosure, the GLP-1 receptor agonist may be in the form of a long-acting conjugate, in which the GLP-1 receptor agonist is linked to an immunoglobulin Fc region, but is not limited thereto. Specifically, the GLP-1 receptor agonist may be one in which imidazo-acetyl exendin-4 (CA exendin-4) is linked to the immunoglobulin Fc region via a linker containing ethylene glycol repeating units, and more specifically, a long-acting conjugate of the GLP-1 receptor agonist, in which one end of the polyethylene glycol linker is linked to lysine (Lys) of the GLP-1 receptor agonist of SEQ ID NO: 72 and the other end of the polyethylene glycol linker is linked to the immunoglobulin Fc region, but is not limited thereto.

In the present disclosure, the GLP-1 receptor agonist may be a GLP-1 receptor agonist efpeglenatide, but is not limited thereto.

Here, the "long-acting conjugate", and the "immunoglobulin Fc region", the "linker containing ethylene glycol repeating units" related thereto are the same as explained above.

The composition of the present disclosure may include any one of the following (i) to (iv):
(i) the glucagon derivative and the GLP-1 receptor agonist;
(ii) the long-acting conjugate of the glucagon derivative and the GLP-1 receptor agonist;
(iii) the glucagon derivative and the long-acting conjugate of the GLP-1 receptor agonist; and
(iv) the long-acting conjugate of the glucagon derivative and the long-acting conjugate of the GLP-1 receptor agonist.

The glucagon derivative or conjugate thereof, and the GLP-1 receptor agonist or conjugate thereof according to the present disclosure; or the composition including the same may exhibit the effects of lowering the blood pressure and/or restoring the renal function in a subject when administered to the subject, but are not limited thereto. Specifically, combined administration of the glucagon derivative or conjugate thereof, and the GLP-1 receptor agonist or conjugate thereof according to the present disclosure may exhibit the effects of lowering the blood pressure; reducing albumin excretion; reducing proteinuria; and/or restoring the renal function, as compared to an excipient control group.

Although not particularly limited, the pharmaceutical composition of the present disclosure may include the glucagon derivative or conjugate thereof, and the GLP-1 receptor agonist or conjugate thereof in an amount of 0.01 % (w/v) to 99% (w/v).

Specifically, the composition of the present disclosure may include the glucagon derivative in an amount of 0.01 nmol/kg to 5 nmol/kg, 0.05 nmol/kg to 4 nmol/kg, 0.05 nmol/kg to 3.5 nmol/kg, or 0.09 nmol/kg to 2.99 nmol/kg, and the GLP-1 receptor agonist in an amount of 0.01 nmol/kg to 5 nmol/kg, 0.05 nmol/kg to 4 nmol/kg, 0.05 nmol/kg to 3.5 nmol/kg, or 0.09 nmol/kg to 2.99 nmol/kg, but is not limited thereto. Further, the composition of the present disclosure may include the glucagon derivative: the GLP-1 receptor agonist at a molar ratio of 0.01 to 50, 0.02 to 40, or 0.03 to 33.22, but is not limited thereto.

With regard to the pharmaceutical composition of the present disclosure, the glucagon derivative or conjugate thereof, and the GLP-1 receptor agonist or conjugate thereof may be administered to a subject in an amount of 0.1 mg to 20 mg, or 0.5 mg to 16 mg, respectively, but are not limited thereto.

The above description may also be applied to other specific embodiments or aspects of the present disclosure, but is not limited thereto.

Still another aspect of the present disclosure provides a method of preventing or treating chronic renal disease, the method including the step of administering, to a subject, the glucagon derivative or conjugate thereof, or the composition including the same.

The method of preventing or treating chronic renal disease according to the present disclosure may further include the step of administering, to a subject, the GLP-1 receptor agonist, the conjugate thereof, or the composition including the same.

Still another aspect of the present disclosure provides a method of preventing or treating chronic renal disease, the method including the step of administering, to a subject, the glucagon derivative or conjugate thereof; and the GLP-1 receptor agonist or conjugate thereof in combination.

The method may be to administer, to a subject, a composition including the glucagon derivative or conjugate thereof, and the GLP-1 receptor agonist or conjugate thereof, or to administer individual preparations of the glucagon derivative or conjugate thereof; and the GLP-1 receptor agonist or conjugate thereof in combination, but is not limited thereto.

The method of the present disclosure may be to administer (i) the glucagon derivative and (ii) the GLP-1 receptor agonist as a single preparation, or to administer the individual preparations simultaneously, individually, sequentially, or in reverse order, but is not limited thereto.

The glucagon derivative, GLP-1 receptor agonist, conjugate thereof, composition, lowering the blood pressure, restoring the renal pressure, chronic renal disease, prevention, and treatment are the same as explained above.

In the present disclosure, the subject refers to mammals including humans, mice, and livestock, and may be a subject having chronic renal disease or at high risk of developing chronic renal disease, but is not limited thereto.

As used herein, the term "administering" refers to introduction of a predetermined material to a patient (subject) by an appropriate manner. The administration route of the glucagon derivative, GLP-1 receptor agonist, conjugate thereof, or composition is not particularly limited, but it may be administered by any general route that enables the delivery of the glucagon derivative, GLP-1 receptor agonist, conjugate thereof, or composition to a target tissue *in vivo,* for example, intraperitoneal, intravenous, intramuscular, subcutaneous, intradermal, oral, topical, intranasal, intrapulmonary, or intrarectal administration.

Further, the glucagon derivative, GLP-1 receptor agonist, conjugate thereof, or composition may be formulated into a single dosage form suitable for administration into the patient's body, and specifically, it is formulated into a preparation useful for peptide drugs according to a common method in the pharmaceutical field, and administered by an oral or parenteral route, such as through skin, intravenously, intramuscularly, intra-arterially, intramedullarily, intrathecally, intraventricularly, pulmonarily, transdermally, subcutaneously, intraperitoneally, intranasally, intragastrically, topically, sublingually, vaginally, or rectally, but is not limited thereto.

Further, the method of the present disclosure may include administering the composition including the glucagon derivative, GLP-1 receptor agonist, or conjugate thereof in a pharmaceutically effective amount. The appropriate total daily dose should be determined within appropriate medical judgment by a physician, and administered once or several times in divided doses. Regarding the objects of the present disclosure, the specific therapeutically effective dose for any particular patient may be preferably applied differently, depending on various factors including the kind and degree of the response to be achieved, specific compositions including whether other agents are occasionally used therewith or not, the patient's age, body weight, general health conditions, sex and diet, the time and route of administration, secretion rate of the composition, duration of treatment, other drugs used in combination or concurrently with the specific composition, and like factors well-known in the medical arts.

The glucagon derivative of the present disclosure, the GLP-1 receptor agonist, the conjugate thereof, or the composition including the same may be, but are not limited to, administered once a week, once every 2 weeks, once every 4 weeks, or once a month, but is not limited thereto.

Specifically, when administered, the glucagon derivative and the GLP-1 receptor agonist may be administered in an amount of about 1 µg to about 200 µg, about 3 µg to about 180 µg, or about 5 µg to about 160 µg per 1 kg of a patient's body weight, and when the two materials are administered in combination, the total dose may be administered in an amount of about 2 µg to about 400 µg, about 6 µg to about 360 µg, or about 10 µg to about 320 µg per 1 kg of a patient's body weight, but is not limited thereto. The glucagon derivative and the GLP-1 receptor agonist to be administered in combination may be administered at a molar ratio of 0.01 to 50, 0.02 to 40, or 0.03 to 33.22, but is not limited thereto.

Specifically, the glucagon derivative may be administered in an amount of 0.09 nmol/kg to 2.99 nmol/kg, and the GLP-1 receptor agonist may be administered in an amount of 0.09 nmol/kg to 2.99 nmol/kg, or the glucagon derivative: the GLP-1 receptor agonist may be administered at a molar ratio of 0.01 to 50, 0.02 to 40, or 0.03 to 33.22, but is not limited thereto.

Still another aspect of the present disclosure provides use of the glucagon derivative or conjugate thereof, or the composition including the same in the prevention or treatment of chronic renal disease.

Still another aspect of the present disclosure provides use of the composition including the glucagon derivative or conjugate thereof and the GLP-1 receptor agonist or conjugate thereof in the prevention or treatment of chronic renal disease.

Still another aspect of the present disclosure provides combined use of the glucagon derivative or conjugate thereof and the GLP-1 receptor agonist or conjugate thereof in the prevention or treatment of chronic renal disease.

The glucagon derivative, GLP-1 receptor agonist, conjugate thereof, composition, lowering the blood pressure, restoring the renal pressure, chronic renal disease, prevention, and treatment are the same as explained above.

Still another aspect of the present disclosure provides use of the glucagon derivative or conjugate thereof, or the composition including the same in the preparation of a medicament for use in the prevention or treatment of chronic renal disease.

Still another aspect of the present disclosure provides use of the composition including the glucagon derivative or conjugate thereof and the GLP-1 receptor agonist or conjugate thereof in the preparation of a medicament for use in the prevention or treatment of chronic renal disease.

The glucagon derivative, GLP-1 receptor agonist, conjugate thereof, composition, lowering the blood pressure, restoring the renal pressure, chronic renal disease, prevention, and treatment are the same as explained above.

Further, still another aspect of the present disclosure provides use of the glucagon derivative or conjugate thereof, or the composition including the same in lowering the blood pressure and/or restoring the renal function.

Still another aspect of the present disclosure provides use of the composition including the glucagon derivative or conjugate thereof and the GLP-1 receptor agonist or conjugate thereof in lowering the blood pressure and/or restoring the renal function.

Still another aspect of the present disclosure provides combined use of the glucagon derivative or conjugate thereof and the GLP-1 receptor agonist or conjugate thereof in lowering the blood pressure and/or restoring the renal function.

The glucagon derivative, GLP-1 receptor agonist, conjugate thereof, composition, lowering the blood pressure, restoring the renal pressure, chronic renal disease, prevention, and treatment are the same as explained above.

Still another aspect of the present disclosure provides use of the glucagon derivative or conjugate thereof, or the composition including the same in the preparation of a medicament for use in lowering the blood pressure and/or restoring the renal function.

Still another aspect of the present disclosure provides use of the composition including the glucagon derivative or conjugate thereof and the GLP-1 receptor agonist or conjugate thereof in the preparation of a medicament for use in lowering the blood pressure and/or restoring the renal function.

The glucagon derivative, GLP-1 receptor agonist, conjugate thereof, composition, lowering the blood pressure, restoring the renal pressure, chronic renal disease, prevention, and treatment are the same as explained above.

Hereinafter, the present disclosure will be described in more detail with reference to the following exemplary embodiments. However, the following exemplary embodiments are only for illustrating the present disclosure, and the scope of the present disclosure is not intended to be limited by these exemplary embodiments.

### Example 1: Preparation of glucagon derivative and measurement of activity

The present inventors intended to examine whether or not the effects of preventing or treating chronic renal disease may be obtained by lowering the blood pressure and restoring the renal function through activity on glucagon receptors.

Accordingly, as described in Table 1 below, a native glucagon and glucagon derivatives having equivalent activity thereto were first prepared. Referring to WO 2016/108586, WO 2017/003191, and WO 2018/004283, the glucagon derivatives were synthesized, and pl and *in vitro* activity thereof were measured.

**[Table 1]**

| Amino acid sequences of native glucagon and glucagon derivatives | | | | |
|---|---|---|---|---|
| SEQ ID NO. | Peptide sequence | Ring formation | pl | *In vitro* activity (Relative Activity to SEQ ID NO: 1, %) |
| SEQ ID NO: 1 | HSQGTFTSDYSKYLDSRRAQDFVQWL MNT | - | 6.8 | 100 |
| SEQ ID NO: 2 | HSQGTFTSDYSKYLDCDRAQDFVQWL MNT | - | 4.56 | 0.6 |
| SEQ ID NO: 3 | HSQGTFTSDYSKYLDCERAQDFVQWL MNT | - | 4.66 | 6.1 |
| SEQ ID NO: 4 | HSQGTFTSDYSKYLDSCDAQDFVQWL MNT | - | 4.13 | < 0.1 |
| SEQ ID NO: 5 | HSQGTFTSDYSKYLDSCEAQDFVQWL MNT | - | 4.22 | 0.3 |
| SEQ ID NO: 6 | HSQGTFTSDYSKYLDSCEADDFVQWLM NT | - | 4.03 | < 0.1 |
| SEQ ID NO: 7 | YSQGTFTSDYSKYLDSCEADDFVQWLM NT | - | 3.71 | < 0.1 |
| SEQ ID NO: 8 | YXQGTFTSDYSKYLDSCDAQDFVQWLI NT | - | 3.77 | < 0.1 |
| SEQ ID NO: 9 | YXQGTFTSDYSKYLDSCDAQDFVVWLI NT | - | 3.77 | < 0.1 |
| SEQ ID NO: 10 | YXQGTFTSDYSKYLDSCDADDFVVWLI NT | - | 3.66 | < 0.1 |
| SEQ ID NO: 11 | YXQGTFTSDYSKYLDEKCAKEFVQWLM NT | - | 4.78 | 4.6 |
| SEQ ID NO: 12 | YXQGTFTSDYSKYLDEKRAKEFVQWLM NTC | ring formed | 6.20 | 56.3 |
| SEQ ID NO: 13 | YXQGTFTSDYSCYLDSRRAQDFVQWL MNT | - | 4.43 | 5.2 |
| SEQ ID NO: 14 | YXQGTFTSDYSKYLDCKRAKEFVQWLM NT | - | 8.12 | 18.1 |
| SEQ ID NO: 15 | YXQGTFTSDYSKYLCEKRAQDFVVWLM NT | - | 6.11 | 1.1 |
| SEQ ID NO: 16 | YXQGTFTSDYSKYLDCRRAQVFVQWL MRT | - | 9.11 | 4.2 |
| SEQ ID NO: 17 | YXQGTFTSDYSKYLDCVRAQDFVQWL MRT | - | 6.03 | 23.2 |
| SEQ ID NO: 18 | YXQGTFTSDYSKYLDSRRACDFRLWLM NT | - | 8.15 | < 0.1 |
| SEQ ID NO: 19 | YXQGTFTSDYSKYLCEKRAKEFVQWLM NT | ring formed | 8.12 | 12.1 |
| SEQ ID NO: 20 | YXQGTFTSDYSKYLDECRAKEFVQWLM | ring formed | 4.78 | 299.7 |
| | NT | | | |
| SEQ ID NO: 21 | YXQGTFTSDYSKYLDEKCAKEFVQWLM NT | ring formed | 4.78 | 57.8 |
| SEQ ID NO: 22 | YXQGTFTSDYSKYLDEKRCKEFVQWLM NT | ring formed | 6.20 | 147.8 |
| SEQ ID NO: 23 | YXQGTFTSDYSKYCDEKRAKEFVQWL MNT | ring formed | 6.20 | 76.8 |
| SEQ ID NO: 24 | YXQGTFTSDYSKCLDEKRAKEFVQWLM NT | ring formed | 6.21 | 58.0 |
| SEQ ID NO: 25 | YXQGTFTSDYSKYLDEKRAKCFVQWLM NT | ring formed | 8.12 | 46.9 |
| SEQ ID NO: 26 | WXQGTFTSDYSKYLDECRAKDFVQWL MNT | ring formed | 4.68 | 1.0 |
| SEQ ID NO: 27 | YXQGTFVSDYSKYLDECRAKDFVQWL MNT | ring formed | 4.68 | 93.6 |
| SEQ ID NO: 28 | WXQGTFVSDYSKYLDECRAKDFVQWL MNT | ring formed | 4.68 | < 0.1 |
| SEQ ID NO: 29 | YXQGTFTSDYSKCLDERRAKDFVQWL MNT | ring formed | 6.15 | 61.3 |
| SEQ ID NO: 30 | WXQGTFTSDYSKCLDERRAKDFVQWL MNT | ring formed | 4.44 | 0.3 |
| SEQ ID NO: 31 | YXQGTFTSDYSKYLDCKRAKEFVQWLM NT | ring formed | 8.12 | 6.3 |
| SEQ ID NO: 32 | - SQGTFTSDYSKYLDECRAKEFVQWLMN T | ring formed | 4.78 | 0.7 |
| SEQ ID NO: 33 | YXQGTFTSDYSKYLDSRRAQDFVQWL MNT | - | 6.04 | 108.2 |
| SEQ ID NO: 34 | WXQGTFTSDYSKYCDERRAKEFVQWL MNT | ring formed | 6.21 | 0.2 |
| SEQ ID NO: 35 | YXQGTFTSDYSKYCDERRAKEFVQWL MNT | ring formed | 6.2 | 17.7 |
| SEQ ID NO: 36 | YXQGTFTSDCSKYLDERRAKEFVQWLM NT | ring formed | 6.21 | 9.9 |
| SEQ ID NO: 37 | YXQGTFTSDYSKYLDERRAKEFVQWLM NTC | ring formed | 6.21 | 225.5 |
| SEQ ID NO: 38 | YXQGTFCSDYSKYLDERRAKEFVQWL MNT | ring formed | 6.15 | 167.3 |
| SEQ ID NO: 39 | YXQGTFVSDCSKYLDERRAKDFVQWL | ring formed | 6.15 | 3.7 |
| | MNT | | | |
| SEQ ID NO: 40 | YXQGTFVSDYSKYLDERRAKDFVQWL MNTC | ring formed | 6.15 | 40.8 |
| SEQ ID NO: 41 | YXQGTFCSDYSKYLDERRAKDFVQWL MNT | ring formed | 6.03 | 45.2 |
| SEQ ID NO: 42 | YXQGTFCSDYSKYLDSRRAQDFVQWL MNT | - | 6.03 | 37.9 |
| SEQ ID NO: 43 | YXQGTFTSDCSKYLDSRRAQDFVQWL MNT | - | 6.03 | 1.6 |
| SEQ ID NO: 44 | YXQGTFTSDYSKYLDSRRAQDFVQWL MNTC | - | 6.21 | 75.4 |
| SEQ ID NO: 45 | YXQGTFTSDYSCYLDEKRAKEFVQWLM NT | - | 4.78 | 5.2 |

In the amino acid sequences described in Table 1, the amino acid represented by X represents aminoisobutyric acid (Aib), which is a non-native amino acid, the underlined amino acids represent formation of a lactam ring between the side chains of the corresponding amino acid pairs underlined and shown in bold, and "-" indicates that no amino acid residue is present on the corresponding position. Further, in the columns with regard to ring formation, "-" indicates that there is no ring formation in the corresponding sequences.

### Example 2: Preparation of long-acting conjugate of glucagon derivative

A long-acting conjugate of the glucagon derivative was prepared by linking the glucagon derivative of SEQ ID NO: 37 prepared in the above Example with an immunoglobulin Fc region using polyethylene glycol as a linker.

Maleimide-PEG-aldehyde (Japanese NOF), which is a linear modified polyethylene glycol with a molecular weight of 10 kDa, in which hydrogens at its two ends were substituted with 3-(3-maleimidopropionamido)propyl group and 3-oxopropyl group (propionaldehyde group), respectively, was reacted with the glucagon derivative of SEQ ID NO: 37, and the cysteine residue of this glucagon derivative peptide was PEGylated at the maleimide end of maleimide-PEG-aldehyde. In detail, the glucagon derivative peptide of SEQ ID NO: 37 and the maleimide-PEG-aldehyde were reacted at a molar ratio of 1:1 to 5 at a protein concentration of 3 mg/mL to 10 mg/mL at low temperature for 1 hour to 3 hours. At this time, the reaction was performed in an environment in which 20% to 60% isopropanol was added to 50 mM Tris buffer (pH 7.5). After the reaction was completed, the reaction solution was applied to SP sepharose HP (GE Healthcare, USA) to purify the glucagon derivative mono-pegylated on cysteine.

Meanwhile, the immunoglobulin Fc region was prepared by using an immunoglobulin Fc region (49.8 kDa, a homodimer prepared by linking two chains of SEQ ID NO: 67 via a disulfide bond) having a hinge region of a Pro-Ser-Cys-Pro sequence (SEQ ID NO: 57) at the N-terminus in accordance with a method described in WO 2007/021129.

Next, the purified mono-pegylated glucagon derivative and the immunoglobulin Fc region were reacted at a molar ratio of 1:2 to 10, at a protein concentration of 10 mg/mL to 50 mg/mL at 4°C to 8°C for 12 hours to 18 hours. The reaction was conducted in an environment in which 10 mM to 50 mM sodium cyanoborohydride as a reducing agent and 10% to 20% isopropanol were added to 100 mM potassium phosphate buffer (pH 6.0). After the reaction was completed, the reaction solution was applied to the butyl sepharose FF purification column (GE Healthcare, USA) and Source ISO purification column (GE Healthcare, USA) to purify a long-acting conjugate of the glucagon derivative peptide, in which the polyethylene glycol end at the aldehyde side of the mono-pegylated glucagon derivative peptide was linked to nitrogen of the N-terminal proline of one chain of the two chains of the immunoglobulin Fc homodimer. The purified conjugate has a structure in which the glucagon derivative, the polyethylene glycol (PEG) linker, and the dimeric immunoglobulin Fc region were covalently linked at a molar ratio of 1:1:1 within the molecule, and the PEG linker was linked to nitrogen of the N-terminal proline of one chain of the two polypeptide chains of the immunoglobulin Fc region.

After preparation, purity was 95% or more, as analyzed by reverse-phase chromatography, size-exclusion chromatography, and ion-exchange chromatography.

In the immunoglobulin Fc region, two monomers having an amino acid sequence of SEQ ID NO: 67 (consisting of 221 amino acids) form a homodimer through a disulfide bond between cysteines, which are the 3^{rd} amino acid of each monomer, and the monomers of the homodimer independently form an intra-disulfide bond between the cysteines at positions 35 and 95 and an intra-disulfide bond between the cysteines at positions 141 and 199, respectively.

Here, the conjugate, in which the glucagon derivative peptide and the immunoglobulin Fc region were linked via PEG, was named a "long-acting conjugate of the glucagon derivative".

### Example 3: Effect of improving renal function in chronic renal disease rat model

In order to investigate the efficacy of improving chronic renal disease (chronic kidney disease, CKD) by the long-acting conjugate of the glucagon derivative prepared in the above Example, a spontaneous hypertensive rat (SHR) model which is known as a hypertension and CKD model was used. SHR is a model in which renal dysfunction is caused by hypertension, and is known to be very similar to that of humans in terms of the etiology of CKD.

First, 16-week-old SHR, which is known to develop renal dysfunction due to blood pressure elevation, was divided into an excipient control group and a group administered with the long-acting conjugate of the glucagon derivative of SEQ ID NO: 37 (3.9 nmol/kg, Q3D, subcutaneous), and repeated administration was performed for 6 weeks. As negative controls, normal rats and excipient control groups were used. To evaluate renal function during 6-week repeated administration, urinary albumin excretion was measured at the time points of day 0, day 13, day 28, and day 46. Statistical analysis was performed using one-way ANOVA (*-***p<0.05-0.001 vs. SHR excipient control group).

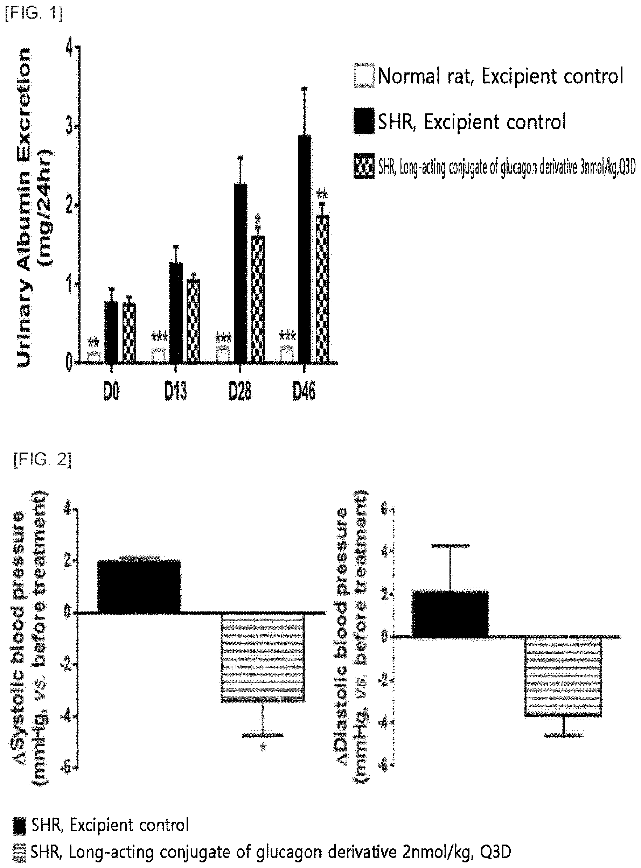

As a result, from 13 days after administration of the long-acting conjugate of the glucagon derivative, urinary albumin excretion began to decrease, as compared to the SHR excipient control group, and it was confirmed that a significant difference appeared after 28 days of administration (FIG. 1).

### Example 4: Effect of lowering blood pressure in chronic renal disease rat model

To investigate the efficacy of lowering the blood pressure in chronic renal disease by the long-acting conjugate of the glucagon derivative of SEQ ID NO: 37 prepared in Example 2, a spontaneous hypertensive rat (SHR) model known as a CKD disease model was used. SHR is a model that is known to show a gradual increase in the blood pressure with age, and is used in evaluating the efficacy of various antihypertensive drugs.

First, a telemetry device was surgically inserted into the aorta of 15-week-old SHR whose blood pressure had risen to the level of hypertension, and then the rats were divided into an excipient control group and a group administered with the long-acting conjugate of the glucagon derivative (2 nmol/kg, Q3D, subcutaneous), and repeated administration was performed for 1 week. Changes in the systolic blood pressure (SBP) and the diastolic blood pressure (DBP) during 1-week repeated administration were monitored every 2 hours through the telemetry device inserted into the aorta. To evaluate the efficacy of lowering the blood pressure by the administered long-acting conjugate of the glucagon derivative of SEQ ID NO: 37, the changes in the mean blood pressure during the administration period of 1 week versus the mean blood pressure for 2 days before administration was calculated, and statistical analysis was performed using t-test (*p<0.05 vs. SHR excipient control group).

As a result, the excipient control group showed the increased blood pressure, as compared to that before administration, whereas the group administered with the long-acting conjugate of the glucagon derivative of SEQ ID NO: 37 showed a decrease in both the systolic and diastolic blood pressures. In particular, it was confirmed that the systolic blood pressure showed a significant difference, as compared to the excipient control group (FIG. 2).

These results confirmed that the long-acting conjugate of the glucagon derivative of the present disclosure also has a therapeutic effect on lowering the blood pressure in chronic renal disease (chronic kidney disease, CKD).

### Example 5: Investigation of effect of improving renal function according to combination administration with GLP-1 receptor agonist

Based on the therapeutic effects of the glucagon derivative of the present disclosure on chronic renal disease, it was intended to further confirm the therapeutic effect on chronic renal disease when a GLP-1 receptor agonist was used in combination with the glucagon derivative of the present disclosure.

### Example 5-1: Preparation of long-acting conjugate of GLP-1 receptor agonist

3.4 kDa propion-ALD2 PEG (IDB, Korea) having propionaldehyde groups at both ends was site-specifically reacted with lysine of GLP-1 receptor agonist (imidazo-acetyl exendin-4 (CA exendin-4)) of SEQ ID NO: 72, and to obtain a conjugate in which PEG and imidazo-acetyl exendin-4 (CA exendin-4) were linked at a ratio of 1:1, the reaction mixture was applied to a cation exchange column to purify mono-PEGylated imidazo-acetyl exendin-4 (CA exendin-4). To prepare an imidazo-acetyl exendin-4 (CA exendin-4)-immunoglobulin Fc region conjugate, in which mono-PEGylated imidazo-acetyl exendin-4 (CA exendin-4) was specifically linked to the N-terminus of the immunoglobulin Fc region, reaction was allowed under a pH condition of 5.0 to 8.2. After the coupling reaction, a two-step purification method using a hydrophobic column and an anion exchange column was used, and finally, the site-specifically linked GLP-1 receptor agonist-immunoglobulin Fc region conjugate was purified.

Here, the conjugate, in which the GLP-1 receptor agonist (imidazo-acetyl exendin-4 (CA exendin-4)) of SEQ ID NO: 72 and the immunoglobulin Fc region were linked through PEG, was named a "long-acting conjugate of GLP-1 receptor agonist" or a "long-acting conjugate of CA-Exd4 derivative".

On the other hand, the immunoglobulin Fc region used in the preparation of the long-acting conjugate of the GLP-1 receptor agonist is the same as the Fc region used in Example 2.

### Example 5-2: Investigation of effect of combination administration with GLP-1 receptor agonist

To investigate the efficacy of improving chronic renal disease (chronic kidney disease, CKD) according to combined administration of the long-acting conjugate of the glucagon derivative of SEQ ID NO: 37 prepared in Example 2 and the long-acting conjugate of the GLP-1 receptor agonist (long-acting conjugate of the CA-Exd4 derivative) prepared in Example 5-1, a spontaneous hypertensive rat (SHR) model which is a CKD disease model was used.

First, 16-week-old SHR, which is known to develop renal dysfunction due to blood pressure elevation, was divided into an excipient control group, a group administered with the long-acting conjugate of the glucagon derivative (3.0 nmol/kg, Q3D, subcutaneous), and a group co-administered with the long-acting conjugate of the glucagon derivative (3.0 nmol/kg, Q3D, subcutaneous) and the long-acting conjugate of the GLP-1 receptor agonist (6.9 nmol/kg, Q3D, subcutaneous), and repeated administration was performed for 10 weeks. As negative controls, normal rats and excipient control groups were used. To evaluate renal function and CKD progression during 10-week repeated administration, urine albumin to creatinine ratio (ACR) was measured at the time points of day 0, day 14, day 28, day 42, day 56, and day 70. Statistical analysis was performed using one-way ANOVA (*-***p<0.05-0.001 vs. SHR excipient control).

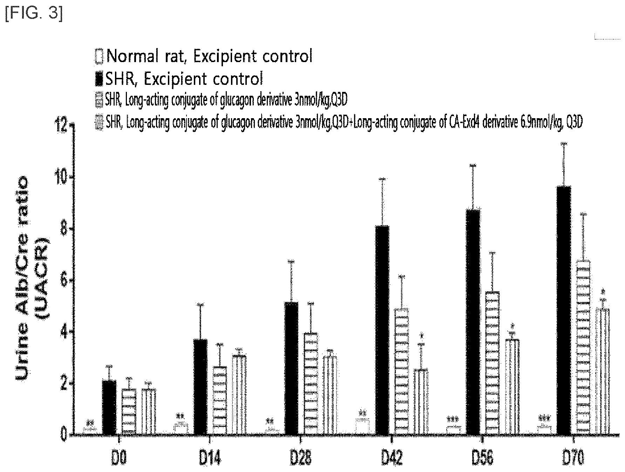

As a result, ACR was continuously elevated in the excipient control group, whereas ACR elevation was delayed in the group administered with the long-acting conjugate of the glucagon derivative. In addition, in the group co-administered with the long-acting conjugate of the glucagon derivative and the long-acting conjugate of the GLP-1 receptor agonist, ACR was significantly reduced, as compared to the excipient control group (FIG. 3).

These results confirmed that the therapeutic efficacy of the long-acting conjugate of glucagon derivative on chronic renal disease (chronic kidney disease, CKD) may be further improved by co-administration with the long-acting conjugate of the GLP-1 receptor agonist.

All of the above experimental results indicate that the glucagon derivative of the present disclosure has useful effects on the treatment of chronic renal disease by the effects of lowering the blood pressure and restoring the renal function, and additional therapeutic effects may also be expected by combined administration with the GLP-1 receptor agonist.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A pharmaceutical composition for preventing or treating chronic renal disease, the pharmaceutical composition comprising a glucagon derivative and a pharmaceutically acceptable excipient,
wherein the glucagon derivative is a peptide including an amino acid sequence of the following General Formula 1: 
X1–X2–QGTF–X7–SD–X10–S–X12–X13–X14–X15–X16–X17–X18–X19–
X20–X21–F–X23–X24–W–L–X27–X28–X29–X30 (General Formula 1, SEQ ID
NO: 46)
wherein X1 is tyrosine (Y);
X2 is alpha-methyl-glutamic acid (α-methyl-glutamic acid), aminoisobutyric acid (Aib), D-alanine, glycine (G), N-methylglycine (Sar), serine (S), or D-serine;
X7 is threonine (T), valine (V), or cysteine (C);
X10 is tyrosine (Y) or cysteine (C);
X12 is lysine (K) or cysteine (C);
X13 is tyrosine (Y) or cysteine (C);
X14 is leucine (L) or cysteine (C);
X15 is aspartic acid (D), glutamic acid (E), or cysteine (C);
X16 is glutamic acid (E), aspartic acid (D), serine (S), *alpha*-methyl-glutamic acid, or cysteine (C), or is absent;
X17 is aspartic acid (D), glutamine (Q), glutamic acid (E), lysine (K), arginine (R), serine (S), cysteine (C), or valine (V), or is absent;
X18 is alanine (A), aspartic acid (D), glutamic acid (E), arginine (R), valine (V), or cysteine (C), or is absent;
X19 is alanine (A), arginine (R), serine (S), valine (V), or cysteine (C), or is absent;
X20 is lysine (K), histidine (H), glutamine (Q), aspartic acid (D), arginine (R), alpha-methyl-glutamic acid, or cysteine (C), or is absent;
X21 is aspartic acid (D), glutamic acid (E), leucine (L), valine (V), or cysteine (C), or is absent;
X23 is isoleucine (I), valine (V), or arginine (R), or is absent;
X24 is valine (V), arginine (R), alanine (A), cysteine (C), glutamic acid (E), lysine (K), glutamine (Q), *alpha*-methyl-glutamic acid, or leucine (L), or is absent;
X27 is isoleucine (I), valine (V), alanine (A), lysine (K), methionine (M), glutamine (Q), or arginine (R), or is absent;
X28 is glutamine (Q), lysine (K), asparagine (N), or arginine (R), or is absent;
X29 is threonine (T); and
X30 is cysteine (C), or is absent
(with the proviso that when the amino acid sequence of General Formula 1 is identical to SEQ ID NO: 1 or SEQ ID NO: 12, it is excluded).

2. The pharmaceutical composition of claim 1, wherein the peptide is in the form of a long-acting conjugate, which is represented by the following Formula 1:
[Formula 1] X-L-F
wherein X represents a peptide including the amino acid sequence of General Formula 1;
L represents a linker containing ethylene glycol repeating units;
F represents an immunoglobulin Fc region; and
- represents covalent linkages between X and L and between L and F, respectively.

3. The pharmaceutical composition of claim 1 or 2, wherein, in General Formula 1,
X2 is aminoisobutyric acid (Aib);
X7 is threonine (T), valine (V), or cysteine (C);
X10 is tyrosine (Y);
X12 is lysine (K);
X13 is tyrosine (Y);
X14 is leucine (L) or cysteine (C);
X15 is aspartic acid (D);
X16 is glutamic acid (E) or serine (S);
X17 is lysine (K), arginine (R), or cysteine (C);
X18 is arginine (R);
X19 is alanine (A) or cysteine (C);
X20 is glutamine (Q) or lysine (K);
X21 is aspartic acid (D) or glutamic acid (E);
X23 is valine (V);
X24 is glutamine (Q);
X27 is methionine (M);
X28 is asparagine (N);
X29 is threonine (T); and
X30 is cysteine (C) or is absent.

4. The pharmaceutical composition of claim 1, wherein the peptide includes an amino acid sequence selected from the group consisting of SEQ ID NOS: 7 to 11, and 13 to 25, 27, 29, 31, 33, and 35 to 45.

5. The pharmaceutical composition of claim 3, wherein the peptide includes an amino acid sequence selected from the group consisting of SEQ ID NOS: 20, 22, 23, 27, 33, 35, 37, 38, 40, 41, 42, and 44.

6. The pharmaceutical composition of claim 1 or 2, wherein the composition exhibits one or more of the following characteristics of:
(a) lowering the blood pressure;
(b) reducing albumin excretion;
(c) reducing proteinuria; and
(d) restoring the renal function.

7. The pharmaceutical composition of claim 1 or 2, wherein the pharmaceutical composition exhibits effects of lowering the blood pressure and restoring the renal function in a subject, when administered to the subject.

8. The pharmaceutical composition of claim 1 or 2, wherein the C-terminus of the peptide is amidated.

9. The pharmaceutical composition of claim 1 or 2, wherein the peptide has a ring formed between amino acid residues.

10. The pharmaceutical composition of claim 2, wherein the immunoglobulin Fc region is aglycosylated.

11. The pharmaceutical composition of claim 2, wherein the immunoglobulin Fc region is an IgG4 Fc region.

12. The pharmaceutical composition of claim 2, wherein the immunoglobulin Fc region is a dimer consisting of two polypeptide chains, and one end of L is linked to only one polypeptide chain of the two polypeptide chains.

13. The pharmaceutical composition of claim 2, wherein, in the long-acting conjugate, L is linked to F and X by covalent linkages formed by reacting one end of L with an amine group or a thiol group of F and by reacting the other end of L with an amine group or a thiol group of X, respectively.

14. The pharmaceutical composition of claim 2, wherein L is polyethylene glycol.

15. The pharmaceutical composition of claim 2, wherein the formula weight of a moiety of the ethylene glycol repeating units in L is in the range of 1 kDa to 100 kDa.

16. The pharmaceutical composition of claim 1 or 2, further comprising a GLP-1 receptor agonist (glucagon-like peptide 1 receptor agonist).

17. The pharmaceutical composition of claim 16, wherein the GLP-1 receptor agonist is selected from the group consisting of GLP-1, exendin-3, exendin-4, agonists thereof, derivatives thereof, fragments thereof, variants thereof, and combinations thereof.

18. The pharmaceutical composition of claim 16, wherein the GLP-1 receptor agonist is a GLP-1 receptor agonist derivative in which the N-terminal histidine residue is substituted with a substance selected from the group consisting of des-amino-histidyl, dimethyl-histidyl, beta-hydroxy imidazopropionyl, 4-imidazoacetyl, and beta-carboxy imidazopropionyl.

19. The pharmaceutical composition of claim 16, wherein the GLP-1 receptor agonist is selected from the group consisting of a native exendin-4, an exendin-4 derivative in which the N-terminal amine group of exendin-4 is deleted, an exendin-4 derivative in which the N-terminal amine group of exendin-4 is substituted with a hydroxyl group, an exendin-4 derivative in which the N-terminal amine group of exendin-4 is modified with a dimethyl group, and an exendin-4 derivative in which the alpha-carbon of a first amino acid (histidine) of exendin-4 is deleted.

20. The pharmaceutical composition of claim 16, wherein the GLP-1 receptor agonist is in the form of a long-acting conjugate, in which the GLP-1 receptor agonist is linked to the immunoglobulin Fc region.

21. The pharmaceutical composition of claim 20, wherein the GLP-1 receptor agonist is an imidazo-acetyl exendin-4 (CA exendin-4) linked to the immunoglobulin Fc region via a linker containing ethylene glycol repeating units.
